(19) **Europäisches Patentamt / European Patent Office / Office européen des brevets**

(11) **EP 3 150 212 B1**

(12) # EUROPEAN PATENT SPECIFICATION

(45) Date of publication and mention of the grant of the patent:
**16.12.2020  Bulletin 2020/51**

(51) Int Cl.:
**A61K 33/00** *(2006.01)*          **A61K 47/04** *(2006.01)*
**A61P 35/00** *(2006.01)*

(21) Application number: **15799410.4**

(22) Date of filing: **25.05.2015**

(86) International application number:
**PCT/CN2015/079686**

(87) International publication number:
**WO 2015/180600 (03.12.2015 Gazette 2015/48)**

(54) **PHARMACEUTICAL SOLUTION HAVING ANTI-TUMOR EFFECT-ENHANCING AND TOXICITY-REDUCING EFFECT, AND PHARMACEUTICAL COMPOSITION COMPRISING SAME**

PHARMAZEUTISCHE LÖSUNG MIT ERHÖHTER WIRKSAMKEIT DER ANTITUMORWIRKUNG UND DER TOXIZITÄTSREDUZIERUNG UND PHARMAZEUTISCHE ZUSAMMENSETZUNG DAMIT

SOLUTION PHARMACEUTIQUE PROMOTRICE DE L'EFFET ANTI-TUMEUR ET AYANT UN EFFET RÉDUCTEUR DE TOXICITÉ, ET COMPOSITION PHARMACEUTIQUE LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2014  CN 201410227737**

(43) Date of publication of application:
**05.04.2017  Bulletin 2017/14**

(73) Proprietor: **Chen, Songyuan**
**Guangzhou, Guangdong 510630 (CN)**

(72) Inventor: **Chen, Songyuan**
**Guangzhou, Guangdong 510630 (CN)**

(74) Representative: **Leifert & Steffan Patentanwälte PartG mbB**
**Patentanwälte**
**Postfach 10 40 09**
**40031 Düsseldorf (DE)**

(56) References cited:
**WO-A1-96/03996        WO-A1-2006/022460**
**CN-A- 102 369 012      US-A1- 2013 142 724**

- **TAKEDA HIROSHI ET AL: "Mechanisms of cytotoxic effects of heavy water (deuterium oxide: D20) on cancer cells", ANTI-CANCER DRUGS, LIPPINCOTT WILLIAMS & WILKINS, US; NL , vol. 9, no. 8 1 September 1998 (1998-09-01), pages 715-725, XP009500770, ISSN: 0959-4973, DOI: 10.1097/00001813-199809000-00007 Retrieved from the Internet: URL:http://epo.summon.serialssolutions.com /2.0.0/link/0/eLvHCXMwtV1dixMxFA0uoiyI6KpY PyAPsijdSDbJNBPfIlXXI9VFpg8-ITSTWYbSztJ2 Xf vvvTeZTqaCog9CGUqShjLn9Pbkzv0g5JW0Snm nPBNS e6a8yVjuhWRcOnNsVWbIFA-K305Vcaa_nmG3h m0Qex r7r0jDGGCNmbP_gHa3KQzAe8AcroA6XP8K93 OPubz1 ah5iNNxm3aybH7XrB26A_f2-Gd5YrI8lArP02Nah vp 4PYWEZctXf**
- **YVONNE B ET AL.: 'Synergistic effects of deuterium oxide and gemcitabine in human pancreatic cancer cell lines' CANCER LETERS vol. 259, no. 2, 08 February 2008, ISSN 0304-3835 pages 231 - 239, XP022392055**
- **HANS J A ET AL.: 'Heavy water enhances the antineoplastic effect o 5-fluoro-uracil and bleomycin in nude mice bearing human carcinoma' INTERNATIONAL JOURNAL OF CANCER vol. 45, no. 3, 15 March 1990, ISSN 0020-7136 pages 475 - 480, XP008059521**

- **JEAN A L ET AL.: 'Survival of tumor-bearing mice exposed to heavy water or heavy water plus methotrexate' CANCER RESEARCH vol. 42, no. 3, 31 March 1982, ISSN 0008-5472 pages 1125 - 1129, XP008059623**
- **David G. Allen* ET AL: "INFLUENCE OF DEUTERIUM OXIDE ON CALCIUM TRANSIENTS AND MYOFIBRILLAR RESPONSES OF FROG SKELETAL MUSCLE", J. Physiol., 1 January 1984 (1984-01-01), pages 225-251, XP55506093, Retrieved from the Internet: URL:https://www.ncbi.nlm.nih.gov/pmc/artic les/PMC1193409/pdf/jphysiol00589-0240.pdf [retrieved on 2018-09-11]**

Remarks:

The file contains technical information submitted after the application was filed and not included in this specification

## Description

## Technical field

The present invention belongs in the field of medicine, and relates to a pharmaceutical solution having an anti-tumor efficacy-enhancing and toxicity-reducing effect.

## Background art

According to a report by the World Health Organization (WHO), in 2014 there were 12 million cases diagnosed with malignancy tumors, and 8.5 million deaths from malignant tumors. Therefore, effective control of malignant tumors is a very urgent and enormous task for people practicing in medicine.

At present, the main therapies against malignant tumors are surgery, radiotherapy, and chemotherapy with various anti-tumor drugs, and these methods have their own characteristics but also certain limitations, especially the unremarkable efficacy often shown in chemotherapy with anti-tumor drugs. For example, a commonly used anti-tumor drug 5-fluorouracil (5-FU) shows an overall response rate of only 10% as a single drug in clinical settings. And many anti-tumor drugs have considerable toxicity which is proportional to their dose. In order to improve efficacy, an increase in dosage of a drug is needed but also results in an increase in toxicity caused by the drug, which poses a dilemma. Therefore, to increase the efficacy of anti-tumor drugs while reducing their toxicity is an important strategy towards improvement of clinical efficacy of anti-tumor drugs.

Many existing anti-tumor drugs must be systemically administered by injection or topically administered by lavage because of their physical or chemical properties, pharmacokinetics and pharmacodynamics. These are commonly used as carrier solutions such as a sodium chloride physiological solution and a 5% glucose solution. These solutions only serve as carriers for anti-tumor drugs, and they neither have any anti-tumor efficacy nor can enhance the efficacy of the anti-tumor drugs.

Deuterium oxide (heavy water, $D_2O$, Mw: 20.03, CAS No. 7789-20-0) can be isolated from seawater. A normal body of adult human contains about 5 g deuterium oxide. It has been demonstrated by experiments using dogs as an animal model that exogenous deuterium oxide that reaches a concentration of 30% in the body causes a toxic effect. Based on this experimental result, theoretically, assuming the body weight of an adult human is 60 kg, deuterium oxide that reaches 18 kg in the body starts to cause toxicity. However, it is actually impossible for a human body to have 18 kg deuterium oxide. Furthermore, the state government of California, USA clearly indicates in the list of chemicals known to the State that deuterium oxide does not cause cancer or defect in reproductive development. Therefore, deuterium oxide should be a non-toxic substance [D.M. Czajka: Am. J. Physiol. 201(1961): 357-362; California Proposition 65]. In 1960s, Gross P.R. *et al.* found that deuterium oxide may inhibit DNA synthase in cells (Science 133(1961): 1131-1133). Afterwards, Laissue *et al.* studied the anti-tumor effect of deuterium oxide using mice as a model (Cancer Res. 42 (1982) 1125-1129), and Altermatt *et al.* studied the anti-tumor effect of drinking deuterium oxide in animals using nude mice bearing a human tumor cells as a model (Cancer 62(1988): 462-466. Int.J. Cancer. 45(1990): 475-480). In the recent 10 years, Hartmann *et al.* demonstrated that deuterium oxide can inhibit *in vitro* proliferation of pancreatic cancer cells (Anticancer Res. 25(2005): 3407-3412), and Bahk *et al.* demonstrated that deuterium oxide can inhibit *in vitro* proliferation of bladder cancer cells (J.Ind. Eng.Chem. 13(2007):501-507).

The aforementioned studies on the anti-tumor effect of deuterium oxide focus on *in vitro* cell-based experiments. It is well acknowledged through research and development of new drugs that drug candidates showing efficacy in *in vitro* cell-based experiments have a chance of only 1 in 100,000 to still show efficacy *in vivo.* Therefore, based on the experimental results using *in vitro* cell culture as a model, whether a drug candidate is clinically efficacious and approvable is poorly predictable. The only two animal studies that have been carried out both use a simple drinking liquid of deuterium oxide, which is a hypotonic solution. The mechanism of action is to increase in the *in vivo* deuterium content to combat tumors. In order to have an effective *in vivo* therapeutic dosage, the animals need to orally take a large dose of the drinking liquid of deuterium oxide. Specifically, in the animal experiments, a drinking liquid of deuterium oxide at a concentration of 30% was prepared by mixing deuterium oxide with drinking water, and used to completely replace drinking water for the animals to drink freely and arbitrarily. Several days to weeks later, the animals reached a deuteration state with a very high level of deuterium, which starts to exert an anti-tumor effect. However, such an drinking liquid of deuterium oxide has serious drawbacks: 1) currently most of the clinically efficacious anti-tumor therapies are a combinational chemotherapy of multiple anti-tumor drugs, while most anti-tumor drugs cannot be orally administered for various reasons; therefore, it is difficult to use deuterium oxide in combination with many anti-tumor drugs, and in turn it is impossible to carry out an effective combinational chemotherapy for patients; 2) the drinking amount of water varies between individuals, and if deuterium oxide is drunk arbitrarily, the dosage therefore cannot be quantified, and the therapeutic effects among different individuals cannot be compared; and under the influence of pharmacokinetic factors like digestion, assimilation, and metabolism, deuterium oxide needs to be taken in a large amount, and is less likely to be approved; 3) the patients need to drink a large amount drinking liquid of deuterium oxide in a short time, which is difficult to implement, results in poor clinical compliance, and also incurs high cost; 4) many mid-stage or late stage cancer patients have dysphagia or have taken a gastrointestinal surgery, and cannot orally take food or water, let alone deuterium oxide.

Moreover, about 50% of mid-stage or late stage cancer patients have tumor metastasis and recurrence in body cavities (such as thoracic cavity, peritoneal cavity, and pelvic cavity), which is the direct cause of death of most patients. Recently developed intrathoracic and intraperitoneal hyperthermic instillation chemotherapy has become an effective therapy in prevention or treatment of intrathoracic and intraperitoneal tumor metastasis and recurrence. However, in this method anti-tumor drugs are dissolved or diluted in the sodium chloride physiological solution and administered by direct (hyperthermic) instillation and lavage in body cavities, and also have the problem of unsatisfactory efficacy. In addition, the sodium chloride physiological solution does not have any anti-tumor efficacy-enhancing action.

Synergistic effects of deuterium oxide and gemcitabine in human pancreatic cancer cell lines have been demonstrated by Bader et al. (Cancer Lett. 259(2008): 231-239). Moreover, WO 2006/022460 A1 discloses compositions and usages of deuterium oxide and compounds containing deuterium oxide for the prevention or suppression of recurrence of urothelial cancer.

Therefore, in the art there is still a demand for a pharmaceutical composition having anti-tumor efficacy, especially a pharmaceutical composition employing a solution that enhances the efficacy.

## Summary of invention

The present invention is defined in the appended claims.

The present invention is made based on the following finding of the inventor:

1) use of deuterium oxide to dissolve or dilute other anti-tumor drugs to prepare various pharmaceutical solutions, for administration by injection or combinational administration by (hyperthermic) instillation and lavage of body cavities;
2) direct injection of small doses of deuterium oxide changes the cell cycle of tumor cells and kills tumor cells;
3) efficacy-enhancing and toxicity reducing effect is obtained by directly dissolving or diluting a small dose of anti-tumors drugs in deuterium oxide, and administering the solution by injection, or by (hyperthermic) instillation and lavage of body cavities, which can achieve the same efficacy as a large dose of the anti-tumor drugs but avoid the toxicity thereof;
4)drug approvability is increased: administration of deuterium oxide by injection allows dosage quantification, which overcomes the drawback of short of drug approvability due to arbitrary drinking without a quantified dose;
5) safety is achieved: administration of a certain dose of deuterium oxide by direct injection, or by (hyperthermic) instillation and lavage of body cavities is safe to mammals.

In summary, deuterium oxide *per se* has anti-tumor efficacy, and when administered in combination with various different anti-tumor drugs, it can significantly enhance the anti-tumor efficacy in a synergistic manner and also reduce toxicity of these anti-tumor drugs, thereby useful for treatment of malignant tumors, which is an unexpected effect.

An aspect of the present invention is to provide a pharmaceutical solution for use according to claim 1.

Further embodiments are disclosed for illustrative purpose.

One aspect of the present disclosure is to provide a pharmaceutical composition, characterized in that the pharmaceutical composition comprises the pharmaceutical solution of the present invention, at least one anti-tumor drug, and optionally one or more pharmaceutically acceptable excipients.

An aspect of the present disclosure is to provide a pharmaceutical solution for use in treating or preventing malignant tumors, characterized by using deuterium oxide as a solvent.

Another aspect of the present disclosure is to provide a pharmaceutical composition for use in treating or preventing malignant tumors, characterized in that the pharmaceutical composition comprises the pharmaceutical solution of the present invention, at least one anti-tumor drug, and optionally one or more pharmaceutically acceptable excipients.

Another aspect of the present disclosure is to provide use of the pharmaceutical solution or the pharmaceutical composition according to the present invention in the manufacture of an anti-tumor agent.

Another aspect of the present disclosure is to provide a medicament for combinational administration, characterized in that the medicament for combinational administration comprises deuterium oxide and at least one anti-tumor drug, wherein the deuterium oxide and the anti-tumor drug may be administered separately, sequentially, or concomitantly.

Another aspect of the present disclosure is to provide a method for preventing or treating malignant tumors, comprising administering a therapeutically effective amount of the pharmaceutical solution, the medicament for combinational administration, or the pharmaceutical composition according to the present invention to a tumor patient.

Another aspect of the present disclosure is to provide use of deuterium oxide in the manufacture of a pharmaceutical solution.

The pharmaceutical solution for use according to the present invention shows a marked anti-tumor effect and lower toxicity than current anti-tumor drugs. The pharmaceutical solution for use according to the present invention is useful for treatment or prevention of malignant tumors. Deuterium oxide shows an effect of synergistically enhancing the anti-tumor efficacy of anti-tumor drugs, such that a small dose of anti-tumor drugs can achieve the same efficacy as a large dose of the anti-tumor drugs, thereby reducing the amount and toxicity of anti-tumor drugs to be used. The pharmaceutical solution for use according to the present invention is useful for preventing or reducing invasion of tumors, drug-resistance of tumors, recurrence of various malignant tumors, such as recurrence, metastasis and implantation of tumor cells in thoracic, peritoneal, bladder and/or pelvic cavities, and malignant effusion in thoracic, peritoneal, bladder and/or pelvic cavities.

**Description of figures**

Figure 1.Effect of deuterium oxide on the cell cycle of human colon cancer HCT-166 cells.

Figure 2.Effect of deuterium oxide on the cell cycle of human colon cancer HCT-166 cells, as measured by the FCM method.

Figure 3.Effect of deuterium oxide on the cell cycle of human lung cancer A549 cells.

Figure 4.Effect of deuterium oxide on the cell cycle of human lung cancer A549 cells, as measured by the FCM method.

Figure 5.Effect of the glucose deuterium oxide solution in combination with 5-FU on the tumor size of xenograft tumors from human colon cancer HCT-166 cells in nude mice.

Figure 6.Effect of the glucose deuterium oxide solution in combination with 5-FU on the tumor weight of xenograft tumors from human colon cancer HCT-166 cells in nude mice.

Figure 7.Effect of the glucose deuterium oxide solution in combination with 5-FU on the body weight of nude mice bearing xenograft tumors from human colon cancer HCT-166 cells.

Figure 8.Effect of the sodium chloride deuterium oxide solution in combination with Gemcitabine on the tumor size of xenograft tumors from human breast cancer MCF-7 cells in nude mice.

Figure 9.Effect of the sodium chloride deuterium oxide solution in combination with Gemcitabine on the tumor weight of xenograft tumors from human breast cancer MCF-7 cells in nude mice.

Figure 10.Effect of the sodium chloride deuterium oxide solution in combination with Gemcitabine on the body weight of nude mice bearing xenograft tumors from human breast cancer MCF-7 cells.

Figure 11.Effect of the sodium chloride deuterium oxide solution in combination with Gemcitabine on the tumor size of xenograft tumors from human lung cancer A549 cells in nude mice.

Figure 12.Effect of the sodium chloride deuterium oxide solution in combination with Gemcitabine on the tumor weight of xenograft tumors from human lung cancer A549 cells in nude mice.

Figure 13.Effect of the sodium chloride deuterium oxide solution in combination with Gemcitabine on the body weight of nude mice bearing xenograft tumors from human lung cancer A549 cells.

In the figures, both DJ and HW refer to deuterium oxide.

**Detailed description of the Disclosure**

The present application discloses the following embodiments.

Embodiment 1. A pharmaceutical solution for use according to claim 1.

Embodiment 2. The pharmaceutical solution according to Embodiment 1, suitable for parenteral administration.

Embodiment 3. The pharmaceutical solution according to Embodiment 1, characterized in that, the pharmaceutical solution comprises sodium chloride, being a sodium chloride deuterium oxide solution containing 0.1 g to 5 g sodium chloride, preferably 0.5 g to 2.5 g sodium chloride per 100 ml pharmaceutical solution.

Embodiment 4. The pharmaceutical solution according to Embodiment 1, characterized in that, the pharmaceutical solution is a sodium chloride deuterium oxide solution containing 0.9 g sodium chloride per 100 ml pharmaceutical solution, which is prepared with deuterium oxide and sodium chloride and has a pH of 4.5 to 7.0.

Embodiment 5. The pharmaceutical solution according to Embodiment 1, characterized in that, the pharmaceutical solution comprises glucose, being a glucose deuterium oxide solution containing 0.1 g to 50 g glucose, preferably 5 to 50 g per 100 ml pharmaceutical solution.

Embodiment 6. The pharmaceutical solution according to Embodiment 1, characterized in that, the pharmaceutical solution is a glucose deuterium oxide solution containing 5 g or 10 g glucose per 100 ml pharmaceutical solution, which is prepared with deuterium oxide and glucose and has a pH of 3.2 to 6.5.

Embodiment 7. The pharmaceutical solution according to Embodiment 1, characterized in that, the pharmaceutical solution is a glucose sodium chloride deuterium oxide solution containing 0.9 g sodium chloride and 5 g glucose per 100 ml pharmaceutical solution, which is prepared with deuterium oxide, sodium chloride and glucose and has a pH of 3.5 to 5.5.

Embodiment 8. The pharmaceutical solution according to Embodiment 1, characterized in that, the pharmaceutical solution is a Ringer's deuterium oxide solution containing 0.9 g sodium chloride, 0.012 g potassium chloride and 0.024 g calcium chloride ($CaCl_2 \cdot 2H_2O$) per 100 ml pharmaceutical solution, which is prepared with deuterium oxide, sodium chloride, potassium chloride and calcium chloride and has a pH of 4.5 to 7.5.

Embodiment 9. The pharmaceutical solution according to Embodiment 1, characterized in that, the pharmaceutical solution is a sodium lactate Ringer's deuterium oxide solution containing 0.310 sodium lactate, 0.600 g sodium chloride, 0.030 g potassium chloride and 0.020 g calcium chloride ($CaCl_2 \cdot 2H_2O$) per 100 ml pharmaceutical solution, which is prepared with deuterium oxide, sodium lactate, sodium chloride, potassium chloride and calcium chloride and has a pH of 6.0 to 7.5.

Embodiment 10. The pharmaceutical solution according to Embodiment 1, characterized in that, the pharmaceutical solution is a composite sodium lactate glucose deuterium oxide solution containing 0.310 sodium lactate, 0.600 g sodium chloride, 0.030 g potassium chloride, 0.020 g calcium chloride and 5 g glucose per 100 ml pharmaceutical solution, which is prepared with deuterium oxide, sodium lactate, glucose, sodium chloride, potassium chloride and calcium chloride and has a pH of 3.5 to 6.5.

Embodiment 11. The pharmaceutical solution according to Embodiment 1, characterized in that, the pharmaceutical solution is a fructose deuterium oxide solution containing 50 g to 100 g fructose per 1000 ml pharmaceutical solution, which is prepared with deuterium oxide and fructose and has a pH of 4.0 to 6.5.

Embodiment 12. The pharmaceutical solution according to Embodiment 1, characterized in that, the pharmaceutical solution is a fructose-sodium chloride deuterium oxide solution containing 50 g fructose and 9 g sodium chloride per 1000 ml pharmaceutical solution, which is prepared with deuterium oxide, fructose and sodium chloride and has a pH of 4.0 to 6.5.

Embodiment 13. The pharmaceutical solution according to Embodiment 1, characterized in that, the pharmaceutical solution is a glycerol fructose sodium chloride deuterium oxide solution containing 100 g glycerol, 50 g fructose and 9 g sodium chloride per 1000 ml pharmaceutical solution, which is prepared with deuterium oxide, glycerol, fructose and sodium chloride and has a pH of 3.0 to 6.0.

Embodiment 14. The pharmaceutical solution according to Embodiment 1, characterized in that, the pharmaceutical solution is a sodium acetate Ringer's deuterium oxide solution containing 1.9 sodium acetate, 3.0 g sodium chloride, 0.15 g potassium chloride and 0.1 g calcium chloride ($CaCl_2 \cdot 2H_2O$) per 500 ml pharmaceutical solution, which is prepared with deuterium oxide, sodium acetate, sodium chloride, potassium chloride and calcium chloride and has a pH of 6.0 to 7.4.

Embodiment 15. The pharmaceutical solution according to Embodiment 1, characterized in that, the pharmaceutical solution is a sodium bicarbonate deuterium oxide solution containing 1 to 50 g, preferably 5 to 10 g, most preferably 5 g sodium bicarbonate per 100 ml pharmaceutical solution, which is prepared with deuterium oxide and sodium bicarbonate and has a pH of 7.5 to 8.5.

Embodiment 16. The pharmaceutical solution according to Embodiment 1, characterized in that, the pharmaceutical solution is a Hank's balanced salt deuterium oxide solution containing 0.8 g sodium chloride, 0.04 g potassium chloride, 0.1 g glucose, 0.006 g potassium dihydrogen phosphate, 0.00475 g disodium hydrogen phosphate, and 0.035 g sodium bicarbonate per 100 ml pharmaceutical solution, which is prepared with deuterium oxide, sodium chloride, potassium chloride, glucose, potassium dihydrogen phosphate, disodium hydrogen phosphate and sodium bicarbonate, and has a pH of 6.0 to 7.5.

Embodiment 17. The pharmaceutical solution according to Embodiment 1, characterized in that, the pharmaceutical solution is an Earle's balanced salt deuterium oxide solution containing 0.8 g sodium chloride, 0.04 g potassium chloride, 0.1 g glucose, 0.006 g potassium dihydrogen phosphate, 0.00475 g disodium hydrogen phosphate, and 0.22 g sodium bicarbonate per 100 ml pharmaceutical solution, which is prepared with deuterium oxide, sodium chloride, potassium chloride, glucose, potassium dihydrogen phosphate, disodium hydrogen phosphate and sodium bicarbonate, and has a pH of 6.0 to 7.5.

Embodiment 18. The pharmaceutical solution according to Embodiment 1, characterized in that, the pharmaceutical solution is a balanced salt deuterium oxide solution containing 0.64 g sodium chloride, 0.075 g potassium chloride, 0.048 g calcium chloride, 0.03 g magnesium chloride, 0.39 g sodium acetate and 0.17 g sodium citrate per 100 ml pharmaceutical solution, which is prepared with deuterium oxide, sodium chloride, potassium chloride, calcium chloride, magnesium chloride, sodium acetate and sodium citrate and has a pH of 6.0 to 7.5.

Embodiment 19. The pharmaceutical solution according to any one of Embodiments 1 to 18, wherein the pharmaceutical solution is a solution for instillation and lavage, a solution for injection, a suspension, an emulsion, or a solution for embolization; and the solution for injection is preferably an intravenous injection, an intra-arterial injection, an intrathecal injection, or an intratumoral and peritumoral injection.

Embodiment 20. The pharmaceutical solution according to Embodiment 19, wherein the dose of the solution for injection is 1 ml/kg to 20 ml/kg.

Embodiment 21. The pharmaceutical solution according to Embodiment 1, wherein the malignant tumor is selected from lung cancer, colorectal cancer, primary liver cancer, esophageal cancer, gastric cancer and cardiac cancer, pancreatic cancer, renal cell carcinoma, bladder cancer, prostate cancer, head and neck cancer, nasopharyngeal cancer, cervical cancer, ovarian cancer, breast cancer, brain tumor, bone and joint sarcoma, thyroid cancer, skin cancer, malignant melanoma, malignant lymphoma, leukemia, and complications and recurrence of various malignant tumors, such as thoracic, peritoneal and/or pelvic cavity metastasis and implantation of tumor cells, and malignant effusion in thoracic, peritoneal and/or pelvic cavity.

Embodiment 22. The method according to Embodiment 21, wherein the lung cancer includes small cell lung cancer (SCLC), non-small cell lung cancer (NSCLC); colorectal cancer includes early stage colorectal cancer, advanced stage colorectal cancer; primary liver cancer include the hepatic cell type, hepatic duct cell type, a mixed type; esophageal cancer includes adenocarcinoma, squamous cell carcinoma, adenosquamous carcinoma, small cell carcinoma; gastric cancer and cardiac cancer include adenocarcinoma, squamous cell carcinoma, adenosquamous carcinoma, small cell carcinoma, malignant gastrointestinal stromal tumor; pancreatic cancer includes ductal cell carcinoma, osteoclast-like giant cell carcinoma; renal cell carcinoma includes clear cell carcinoma and papillary renal cell carcinoma; bladder cancer includes urothelial carcinoma, squamous cell carcinoma, adenocarcinoma; prostate cancer includes adenocarcinoma, ductal adenocarcinoma, urothelial carcinoma, and squamous cell carcinoma; head and neck cancer includes squamous cell carcinoma, adenocarcinoma, and adenosquamous carcinoma; nasopharyngeal carcinoma includes squamous cell carcinoma and adenocarcinoma; cervical cancer includes squamous cell carcinoma, adenocarcinoma, and sarcoma; ovarian cancer includes ovarian epithelial cancer, germ cell tumors, and ovarian sex cord-stromal tumors; breast cancer includes epithelial tumors, mesenchymal tumors, and myoepitheliomas; brain tumors include primary brain tumors and brain tumor metastases; bone and joint sarcomas includes chondrosarcoma, osteosarcoma, Ewing's sarcoma, and soft tissue sarcoma; thyroid cancer includes papillary carcinoma, follicular carcinoma, and medullary carcinoma; skin cancer includes basal cell carcinoma and squamous cell carcinoma; malignant melanoma includes superficial diffusive melanoma, nodular melanoma, and acral-lentiginous melanoma; malignant lymphomas include Hodgkin's lymphoma and non-Hodgkin's lymphoma; leukemia includes acute leukemia and chronic leukemia.

In the pharmaceutical solution according to the present invention, every 100 ml of the pharmaceutical solution contains 1 to 99.9 ml deuterium oxide, preferably 9 to 99.9 ml deuterium oxide, more preferably 20 to 99.9 ml deuterium oxide, even more preferably 30 to 99.9 ml deuterium oxide, even more preferably 40 to 99.9 ml deuterium oxide, even more preferably 50 to 99.9 ml deuterium oxide, even more preferably 60 to 99.9 ml deuterium oxide, even more preferably 70 to 99.9 ml deuterium oxide, even more preferably 80 to 99.9 ml deuterium oxide, even more preferably 90 to 99.9 ml deuterium oxide, and most preferably 95 to 99.9 ml deuterium oxide.

Deuterium oxide used in the present invention is produced by deuterium oxide plants, and is commercially available. In the deuterium oxide the isotope abundance of deuterium is 1 to 99.9%, preferably 30 to 99.9%, more preferably 50 to 99.9%, more preferably 70 to 99.9%, and most preferably 90 to 99.9%. For example the deuterium oxide provided by Cambridge Isotope Laboratories, Inc. USA, may be used, which has an isotope abundance of deuterium of 90 to 99.9% (D 90-99.9%).

The pharmaceutically excipients may be water, sodium chloride, potassium chloride, calcium chloride, magnesium chloride, sodium lactate, sodium acetate, sodium citrate, sodium bicarbonate, potassium dihydrogen phosphate, disodium hydrogenphosphate, sodium bicarbonate, glucose, fructose, albumin, liposomes, hyaluronic acid, and/or polyethylene glycol.

The anti-tumor drug can be selected from anti-tumor cytotoxic drugs and monoclonal antibody anti-tumor drugs.

The anti-tumor cytotoxic drugs include antimetabolites, phytogenic anti-tumor drugs, tumor antibiotics, alkylating agents, monoclonal antibodies, and platinum preparations. The antimetabolites include 5-fluorouracil, gemcitabine, floxuridine, pemetrexed, raltitrexed, fludarabine, cytarabine; preferably 5-fluorouracil, gemcitabine, and pemetrexed. The tumor antibiotics include mitomycin, epirubicin, peplomycin, daunorubicin, adriamycin, pirarubicin, aclarubicin; preferably mitomycin and epirubicin. The platinum preparations include cisplatin, oxaliplatin, carboplatin, nedaplatin; preferably cisplatin and oxaliplatin. The phytogenic anti-tumor drugs include paclitaxel, paclitaxel liposomes, paclitaxel albumin, docetaxel, etoposide, hydroxycamptothecin; preferably paclitaxel, paclitaxel liposomes, paclitaxel albumin, and docetaxel. The alkylating agents include thiotepa, carmustine, nimustine, fotemustine, estramustine, cyclophosphamide, myleran; preferably thiotepa and carmustine.

The monoclonal antibody anti-tumor drugs include bevacizumab, cetuximab, trastuzumab, panitumumab, nimotuzumab, recombinant human endostatin; preferably bevacizumab and recombinant human endostatin.

However, the inventively used anti-tumor drug is selected from the group consisting of 5-fluorouracil, gemcitabine, mitomycin, oxaliplatin, paclitaxel, thiotepa and bevacizumab.

The pharmaceutical solution for use according to the present invention is prepared by standard methods for preparing solution in the field of pharmaceutics, and satisfys relevant standards in *Chinese Pharmacopoeia, United States Pharmacopoeia,* and *European Pharmacopoeia.*

When the pharmaceutical solution is combined with anti-tumor drugs, the dissolution or dilution ratios of anti-tumor drugs in the pharmaceutical solution prescribed in *Chinese Pharmacopoeia, United States Pharmacopoeia,* and *European Pharmacopoeia* are used.

The components in the pharmaceutical solution may be administered separately, sequentially, or concomitantly.

The pharmaceutical solution for use according to the present invention is various preparations such as a solution for instillation and lavage, a solution for injection, a suspension, an emulsion, or a solution for embolization, preferably a solution for instillation and lavage and a solution for injection.

The pharmaceutical solution for use according to the present invention is suitable for administrations by (hyperthermic) instillation and lavage of body cavities, intravenous injection, intra-arterial injection, intrathecal injection, and intratumoral and peritumoral injection, preferably administrations by (hyperthermic) instillation and lavage of body cavities and by intravenous injection. The dose selected for administration by intravenous injection is 1 ml/kg to 20 ml/kg.

The malignant tumor is selected from lung cancer, colorectal cancer, primary liver cancer, esophageal cancer, gastric cancer and cardiac cancer, pancreatic cancer, renal cell carcinoma, bladder cancer, prostate cancer, head and neck cancer, nasopharyngeal cancer, cervical cancer, ovarian cancer, breast cancer, brain tumor, bone and joint sarcoma, thyroid cancer, skin cancer, malignant melanoma, malignant lymphoma, leukemia, and complications and recurrence of various malignant tumors, such as thoracic, peritoneal and/or pelvic cavity metastasis and implantation of tumor cells, and malignant effusion in thoracic, peritoneal and/or pelvic cavity.

Lung cancer includes small cell lung cancer (SCLC), non-small cell lung cancer and the like; colorectal cancer includes early stage colorectal cancer, advanced stage colorectal cancer, and the like; primary liver cancer include the hepatic cell type, hepatic duct cell type, a mixed type, and the like; esophageal cancer includes adenocarcinoma, squamous cell carcinoma, adenosquamous carcinoma, small cell carcinoma, and the like; gastric cancer and cardiac cancer include adenocarcinoma, squamous cell carcinoma, adenosquamous carcinoma, small cell carcinoma, malignant gastrointestinal stromal tumor, and the like; pancreatic cancer includes ductal cell carcinoma, osteoclast-like giant cell carcinoma, and the like; renal cell carcinoma includes clear cell carcinoma and papillary renal cell carcinoma; bladder cancer includes urothelial carcinoma, squamous cell carcinoma, adenocarcinoma, and the like; prostate cancer includes adenocarcinoma, ductal adenocarcinoma, urothelial carcinoma, and squamous cell carcinoma; head and neck cancer includes squamous cell carcinoma, adenocarcinoma, and adenosquamous carcinoma; nasopharyngeal carcinoma includes squamous cell carcinoma and adenocarcinoma; cervical cancer includes squamous cell carcinoma, adenocarcinoma, and sarcoma; ovarian cancer includes ovarian epithelial cancer, germ cell tumors, and ovarian sex cord-stromal tumors; breast cancer includes epithelial tumors, mesenchymal tumors, and myoepitheliomas; brain tumors include primary brain tumors and brain tumor metastases; bone and joint sarcomas includes chondrosarcoma, osteosarcoma, Ewing's sarcoma, and soft tissue sarcoma; thyroid cancer includes papillary carcinoma, follicular carcinoma, and medullary carcinoma; skin cancer includes basal cell carcinoma and squamous cell carcinoma; malignant melanoma includes superficial diffusive melanoma, nodular melanoma, and acral-lentiginous melanoma; malignant lymphomas include Hodgkin's lymphoma and non-Hodgkin's lymphoma; leukemia includes acute leukemia and chronic leukemia.

The word "comprise" or "comprising" used herein encompasses both the case where the substance(s) or component(s) referred to is exclusively included, and the case where other substance(s) or component(s) that does not interfere with the accomplishment of the intended objective is also included in addition to the substance or component referred to.

Unless otherwise specified, all values used herein to indicate the amount of a substance should be construed as being modified by the term "approximately".

The present invention will be further described below in conjunction with Examples. It is to be understood that the scope of the present invention is not limited to the Examples.

It is also to be understood, that examples not falling into the scope of the claims are to be regarded as reference examples only.

All cell lines used in the Examples of the present application are commercially available.

**Example 1**

0.9 g sodium chloride was weighed out, to which 99 ml deuterium oxide (D, 99.8%, from Cambridge Isotope Laboratories, Inc., USA, the same below) was added to dissolve the sodium chloride under stirring. Drops of a sodium hydroxide solution were added to adjust the solution pH to 7.0, and then deuterium oxide was added to a final volume of 100 ml. The solution was filtered through a millipore filter and sealed in a container, followed by sterilization and disinfection, to obtain a sodium chloride deuterium oxide solution containing 0.9 g sodium chloride per 100 ml pharmaceutical solution.

**Example 2**

5 g glucose was weighed out, to which 99 ml deuterium oxide was added to dissolve the glucose under stirring. Drops of a hydrochloric acid solution were added to adjust the pH to 5.5, and then deuterium oxide was added to a final volume of 100 ml. The solution was filtered through a millipore filter and sealed in a container, followed by sterilization and disinfection, to obtain a glucose deuterium oxide solution containing 5 g glucose per 100 ml pharmaceutical solution.

**Example 3**

10 g glucose was weighed out, to which 99 ml deuterium oxide was added to dissolve the glucose under stirring. Drops of a hydrochloric acid solution were added to adjust the pH to 5.5, and then deuterium oxide was added to a final volume of 100 ml. The solution was filtered through a millipore filter and sealed in a container, followed by sterilization and disinfection, to obtain a glucose deuterium oxide solution containing 10 g glucose per 100 ml pharmaceutical solution.

**Example 4**

0.9 g sodium chloride and 5 g glucose were separately weighed out, to which 99 m deuterium oxide was added to dissolve them under stirring. Drops of a hydrochloric acid solution were added to adjust the pH to 5.0, and then deuterium oxide was added to a final volume of 100 ml. The solution was filtered through a millipore filter, sealed in a container, followed by sterilization and disinfection, to obtain a glucose sodium chloride deuterium oxide solution containing 0.9 g sodium chloride and 5 g glucose per 100 ml pharmaceutical solution.

### Example 5

0.85 g sodium chloride, 0.03 g potassium chloride and 0.033 g calcium chloride ($CaCl_2 \cdot 2H_2O$) were separately weighed out, to which 99 ml deuterium oxide was added to dissolve them under stirring. Drops of a sodium hydroxide solution were added to adjust the pH to 7.0, and then deuterium oxide was added to a final volume of 100 ml. The solution was filtered through a millipore filter and sealed in a container, followed by sterilization and disinfection, to obtain a Ringer's deuterium oxide solution.

### Example 6

0.31 g sodium lactate, 0.6 g sodium chloride, 0.03 g potassium chloride and 0.02 g calcium chloride ($CaCl_2 \cdot 2H_2O$) were separately weighed out, to which 99 ml deuterium oxide was added to dissolve them under stirring. Drops of a sodium hydroxide solution were added to adjust the pH to 7.0, and then deuterium oxide was added to a final volume of 100 ml. The solution was filtered through a millipore filter, and sealed in a container, followed by sterilization and disinfection, to obtain a sodium lactate Ringer's deuterium oxide solution.

### Example 7

0.31 sodium lactate, 0.6 g sodium chloride, 0.03 g potassium chloride, 0.02 g calcium chloride ($CaCl_2 \cdot 2H_2O$) and 5 g glucose were separately weighed out, to which 99 ml deuterium oxide was added to dissolve them under stirring. Drops of a hydrochloric acid solution were added to adjust the pH to 6.0, and then deuterium oxide was added to a final volume of 100 ml. The solution was filtered through a millipore filter, and sealed in a container, followed by sterilization and disinfection, to obtain a composite sodium lactate glucose Ringer's deuterium oxide solution.

### Example 8

25 g or 50 g fructose was weighed out, to which 499 ml deuterium oxide was added to dissolve it under stirring. Drops of a hydrochloric acid solution were added to adjust the pH to 6.0, and then deuterium oxide was added to a final volume of 500 ml. The solution was filtered through a millipore filter, and sealed in a container, followed by sterilization and disinfection, to obtain a fructose deuterium oxide solution containing 25 g or 50 g fructose per 500 ml pharmaceutical solution.

### Example 9

12.5 g fructose and 2.25 g sodium chloride were separately weighed out, to which 249 ml deuterium oxide was added to dissolve them under stirring. Drops of a hydrochloric acid solution were added to adjust the pH to 6.0, and then deuterium oxide was added to a final volume of 250 ml. The solution was filtered through a millipore filter, and sealed in a container, followed by sterilization and disinfection, to obtain a fructose sodium chloride deuterium oxide solution.

### Example 10

100 g glycerol, 50 g fructose and 9 g sodium chloride were separately weighed out, to which 999 ml deuterium oxide was added to dissolve them under stirring. Drops of a hydrochloric acid solution were added to adjust the pH to 6.0, and then deuterium oxide was added to a final volume of 1000 ml. The solution was filtered through a millipore filter, and sealed in a container, followed by sterilization and disinfection, to obtain a glycerol fructose sodium chloride deuterium oxide solution.

### Example 11

1.9 sodium acetate, 3.0 g sodium chloride, 0.15 g potassium chloride and 0.1 g calcium chloride were separately weighed out, to which 499 ml deuterium oxide was added to dissolve them under stirring. Drops of a sodium hydroxide solution were added to adjust the pH to 7.0, and then deuterium oxide was added to a final volume of 500 ml. The solution was filtered through a millipore filter, and sealed in a container, followed by sterilization and disinfection, to obtain a composite sodium acetate deuterium oxide solution.

### Example 12

5 g or 10 g sodium bicarbonate was weighed out, to which 99 ml deuterium oxide was added to dissolve it under stirring. Drops of a sodium hydroxide solution were added to adjust the pH to 8.0, and then deuterium oxide was added to a final volume of 100 ml. The solution was filtered through a millipore filter, and sealed in a container, followed by sterilization and disinfection, to obtain a sodium bicarbonate deuterium oxide solution containing 5 g or 10 g sodium bicarbonate per 100 ml pharmaceutical solution.

**Example 13**

0.8 g sodium chloride, 0.04 g potassium chloride, 0.1 g glucose, 0.006 g potassium dihydrogen phosphate, 0.00475 g disodium hydrogen phosphate, and 0.035 g sodium bicarbonate were separately weighed out, to which 99 ml deuterium oxide was added to dissolve them under stirring. Drops of a sodium hydroxide solution were added to adjust the pH to 7.0, and then deuterium oxide was added to a final volume of 100 ml. The solution was filtered through a millipore filter, and sealed in a container, followed by sterilization and disinfection, to obtain a Hank's balanced salt deuterium oxide solution.

**Example 14**

0.8 g sodium chloride, 0.04 g potassium chloride, 0.1 g glucose, 0.006 g potassium dihydrogen phosphate, 0.00475 g disodium hydrogen phosphate and 0.22 g sodium bicarbonate were separately weighed out, to which 99 ml deuterium oxide was added to dissolve them under stirring. Drops of a sodium hydroxide solution were added to adjust the pH to 7.0, and then deuterium oxide was added to a final volume of 100 ml. The solution was filtered through a millipore filter, and sealed in a container, followed by sterilization and disinfection, to obtain an Earle's balanced salt deuterium oxide solution.

**Example 15**

0.64 g sodium chloride, 0.075 g potassium chloride, 0.048 g calcium chloride, 0.03 g magnesium chloride, 0.39 g sodium acetate and 0.17 g sodium citrate were separately weighed out, to which 99 ml deuterium oxide was added to dissolve them under stirring. Drops of a sodium hydroxide solution were added to adjust the pH to 7.0, and then deuterium oxide was added to a final volume of 100 ml. The solution was filtered through a millipore filter, and sealed in a container, followed by sterilization and disinfection, to obtain a composite balanced salt deuterium oxide solution.

**Example 16**

Studies of *in vitro* inhibition of growth of human tumor cells by deuterium oxide, by the MTT (3-(4,5-dimethylthiazol-2-yl)-2,5-diphenyltetrazolium bromide) assay
Cell line: all cell lines of various types shown in Table 1 were provided by NANJING KEYGEN BIOTECH. CO., LTD and from ATCC, and were commercially available (the same below).

Method:

1. A bottle of cells that had been cultured for 3 to 4 days and were in the exponential phase was taken, a suitable amount of 0.25% Trypsin-EDTA solution was added thereto to detach the cells from the wall, and the cells were suspended in 10 ml RPMI 1640 medium containing 10% FBS.
2. The cells were counted on a hemocytometer, and generally viable cells should be not less than 97%.
3. The cell suspension was diluted with a complete medium to prepare a suspension containing $1 \times 10^4$ cells/ml.
4. To each well of a 12-well culture plate, 300 $\mu$l cell suspension was added. The addition of cells should be finished within 4 hours. The plate was placed in an incubator at 37°C and 5% $CO_2$ for 24 hours.
5. Powdery RPMI Medium 1640 (Gibco, USA, Cat#: 31800-022) was dissolved in deuterium oxide (provided by Cambridge Isotope Laboratories, Inc. (US)) to prepare a cell culture medium (unless particularly specified, all culture media used in the Examples hereinafter were prepared from powdery RPMI Medium 1640 and deuterium oxide) at a concentration shown in Table 1, which was added into the cell culture plate.
6. The cell culture plate was incubated for 3 days in an incubator at 37°C, 5% $CO_2$ and 100% humidity.
7. A solution of 1 mg/ml MTT was prepared in a serum-free RPMI1640 medium, added to the plate at 200 $\mu$l/well, and incubated at 37°C for 4 hours, to allow MTT to be reduced to formazan.
8. The supernatant was removed by pipetting, 200 $\mu$l DMSO was added to dissolve formazan, and the solution was well mixed on a platform shaker.

9. Absorbance of each well was measured on an ELISA reader with a detection wavelength of 570 nm and a reference wavelength of 450 nm.

10. The assay was repeated one more time.

Calculation of results: Inhibition of growth of tumor cells by deuterium oxide was calculated using tumor cells treated with physiological saline as a control group. Inhibition of cell growth was plotted against amounts of deuterium oxide to obtain a dose response curve, from which the half maximal inhibitory concentration ($IC_{50}$) of deuterium oxide was derived.

$$\text{Inhibition of tumor cell growth } (\%) = (1 - \text{OD test/OD control}) \times 100\%$$

Table 1 (a-1). *In vitro* inhibition of growth of human tumor cells by deuterium oxide

| Deuteri um oxide conc. (v/v) | Inhibition of growth (%) | | | | | | | |
|---|---|---|---|---|---|---|---|---|
| | Lung cancer cell line | | | Pancreatic cancer cell line | | | Colon cancer cell line | Gastric cancer cell line |
| | A549 | NCI-H460 | NCI-1975 | CFPAC-1 | ASPC-1 | PANC-1 | HCT-116 | BGC-823 |
| 80% | 92.34 | 98.06 | 87.23 | 91.85 | 85.83 | 79.05 | 94.62 | 74.57 |
| 70% | 70.88 | 82.33 | 79.65 | 77.94 | 70.14 | 57.29 | 70.42 | 61.90 |
| 60% | 55.42 | 69.50 | 51.93 | 61.42 | 52.22 | 50.31 | 66.31 | 37.83 |
| 50% | 42.67 | 53.09 | 39.10 | 45.30 | 44.14 | 31.62 | 48.43 | 15.99 |
| 40% | 39.37 | 42.05 | 24.03 | 33.67 | 23.99 | 26.73 | 37.43 | 7.50 |
| 30% | 27.71 | 30.58 | 6.66 | 26.16 | 9.70 | 17.81 | 30.21 | 12.32 |
| 20% | 16.19 | 11.60 | 5.60 | 14.84 | -7.26 | 9.06 | 23.32 | 9.09 |
| 10% | 18.88 | 9.47 | -4.95 | 10.75 | -4.88 | 8.43 | 18.58 | -3.60 |
| 5% | 18.05 | 11.27 | -7.44 | 13.42 | -0.20 | 14.41 | 21.64 | -4.23 |
| 2.5% | 17.64 | 9.40 | -8.29 | 14.05 | 6.35 | 13.88 | 31.55 | 2.15 |
| 1.2% | 7.71 | 1.15 | 0.84 | 6.82 | 0.64 | 7.37 | 11.81 | 2.74 |

Table 1 (a-2). *In vitro* inhibition of growth of human tumor cells by deuterium oxide

| Deuteri umoxide conc. (v/v) | Inhibition of growth (%) | | | | | | | |
|---|---|---|---|---|---|---|---|---|
| | Cervical cancer cell line | Breast cancer cell line | Bladder cancer cell line | Ovarian cancer cell line | Prostate cancer cell line | Brain cancer cell line | Osteosarcoma cell line | Leukemia cell line |
| | HeLa | MCF-7 | 5637 | SKOV3 | PC-3 | SF767 | U2-OS | K562 |
| 80% | 90.11 | 98.08 | 88.43 | 90.42 | 87.08 | 71.22 | 45.84 | 93.24 |
| 70% | 85.82 | 89.06 | 74.86 | 74.07 | 73.42 | 49.65 | 29.64 | 88.59 |
| 60% | 73.32 | 86.41 | 59.76 | 59.84 | 60.84 | 4053 | 23.43 | 70.13 |
| 50% | 65.63 | 63.22 | 52.11 | 41.80 | 59.42 | 21.23 | 18.21 | 55.51 |
| 40% | 58.26 | 52.45 | 44.32 | 32.76 | 43.19 | 16.50 | 10.23 | 39.51 |
| 30% | 45.41 | 40.38 | 36.69 | 16.69 | 26.67 | 8.42 | 11.99 | 22.72 |
| 20% | 36.12 | 31.60 | 25.56 | 12.64 | -16.46 | 7.66 | -3.12 | 9.10 |
| 10% | 28.87 | 22.47 | 16.15 | 9.65 | 8.81 | -8.43 | -8.55 | -7.60 |

(continued)

| Deuterium oxide conc. (v/v) | Inhibition of growth (%) | | | | | | | |
|---|---|---|---|---|---|---|---|---|
| | Cervical cancer cell line | Breast cancer cell line | Bladder cancer cell line | Ovarian cancer cell line | Prostate cancer cell line | Brain cancer cell line | Osteosarcoma cell line | Leukemia cell line |
| | HeLa | MCF-7 | 5637 | SKOV3 | PC-3 | SF767 | U2-OS | K562 |
| 5% | 16.25 | 15.71 | 10.44 | 8.33 | -2.50 | -4.41 | -1.64 | 4.23 |
| 2.5% | 14.44 | 11.04 | -3.09 | 7.35 | 4.33 | 3.94 | 4.20 | 6.51 |
| 1.2% | 12.11 | 9.78 | 0.84 | 1.82 | 0.46 | 4.76 | -2.81 | -2.47 |

Table 1 (b) shows $IC_{50}$ and $IC_{10}$ (expressed in v/v%) of deuterium oxide against different types tumor cells.

Table 1 (b-1). $IC_{50}$ and $IC_{10}$ for *in vitro* inhibition of growth of human tumor cells by deuterium oxide

| | Lung cancer cell line | | | Pancreatic cancer cell line | | | Colon cancer cell line | Gastric cancer cell line |
|---|---|---|---|---|---|---|---|---|
| | A549 | NCI-H460 | NCI-1975 | CFPAC-1 | ASPC-1 | PANC-1 | HCT-116 | BGC-823 |
| $IC_{50}$ | 55.32% | 48.75% | 62.61% | 59.43% | 57.00% | 65.38% | 51.13% | 96.68% |
| $IC_{10}$ | 1.52% | 5.60% | 27.00% | 3.20% | 11.80% | 3.03% | 0.25% | 26.92% |

Table 1 (b-2). $IC_{50}$ and $IC_{10}$ for *in vitro* inhibition of growth of human tumor cells by deuterium oxide

| | Cervical cancer cell line | Breast cancer cell line | Bladder cancer cell line | Ovarian cancer cell line | Pro state cancer cell line | Brain cancer cell line | Osteosarcoma cell line | Leukemia cell line |
|---|---|---|---|---|---|---|---|---|
| | HeLa | MCF-7 | 5637 | Caov-3 | PC-3 | SF767 | U2-OS | K562 |
| $IC_{50}$ | 68.33% | 61.29% | 59.08% | 46.83% | 61.34% | 91.12% | 86.25% | 51.72% |
| $IC_{10}$ | 1.8% | 3.28% | 4.46% | 11.51% | 9.35% | 31.85% | 24.99% | 14.83% |

It can be seen from Table 17(b) that deuterium oxide shows different $IC_{50}$ and $IC_{10}$ against different types of tumor cells.

The results demonstrate that deuterium oxide shows a certain inhibitory effect on the growth of various cancer cell lines, and higher contents of deuterium oxide show more significant inhibition of growth of tumor cells.

**Example 17**

*In vitro* inhibition of growth of human colon cancer HCT-116 cells (a moderately drug resistant strain) by deuterium oxide in combination with 5-fluorouracil (5-FU)

Cell lines: human colon cancer cells HCT-116 were provided by NANJING KEYGEN BIOTECH. CO., LTD.

Test drugs: 5-fluorouracil (5-FU), and the sodium chloride deuterium oxide solution prepared in Example 1.

Method: 5-fluorouracil was diluted with the sodium chloride deuterium oxide solution according to the concentrations shown in Table 2 and added to a cell culture plate; the *in vitro* inhibition of tumor cell growth was measured by the MTT assay in the same manner as Example 16.

The assay was repeated one more time.

Result: it was found that 5-FU showed a certain inhibitory effect on growth of human colon cancer HCT-116 cells (a moderately drug resistant strain), and can significantly enhance the anti-tumor efficacy when combined with deuterium oxide, as shown in Table 2.

Table 2. Inhibition of growth of human colon cancer HCT-116 cells (a moderately drug resistant strain) by deuterium oxide in combination with 5-FU

| 5-FU Final conc. (μg/ml) | Inhibition of cell growth (%) (5-FU alone) | Inhibition of cell growth (%) [5-FU + 10% Deuterium oxide (v/v)] |
|---|---|---|
| 12.5 | 35.32 | 80.51 |
| 6.25 | 26.52 | 67.15 |
| 3.13 | 18.58 | 62.24 |
| 1.56 | 11.04 | 51.15 |
| 0.78 | 9.82 | 45.36 |
| 0.39 | 6.98 | 32.94 |
| 0.20 | 3.24 | 28.82 |
| 0.10 | 2.30 | 16.45 |
| 0.05 | 2.55 | 11.14 |
| 0.02 | 2.18 | 10.76 |

The results demonstrate that the combination of deuterium oxide and 5-FU enhances the inhibitory effect on growth of drug-resistant tumor cells (a moderately drug resistant strain); and especially at a low concentration of 5-FU, deuterium oxide shows a more significant efficacy-enhancing effect. Therefore, deuterium oxide has a sensitizing effect against drug resistance.

**Example 18**

Inhibition of invasion of human colon cancer HCT-116 cells and human lung cancer A549 cells by deuterium oxide in combination with 5-fluorouracil or gemcitabine (by the Transwell method)
Cell lines: human colon cancer HCT-116 cells and human lung cancer A549 cells were provided by NANJING KEYGEN BIOTECH. CO., LTD.
Test drugs: gemcitabine (GEM), 5-fluorouracil (5-FU), and the sodium chloride deuterium oxide solution prepared in Example 1.

Method:

Cell culturing

1. Cell resuscitation

1.1. Warm water at 37°C to 40°C was prepared, a cryogenic vial was taken out of liquid nitrogen and immediately put into the 37°C-40°C warm water, followed by vigorous shaking until the cryogenic liquid was completely thawed;
1.2. The suspension of cryogenic cells was transferred to a centrifugal tube, 5 ml culture medium was added thereto, and the cells were well mixed in the medium by gentle pipetting;
1.3. The cell suspension was centrifuged at 800 to 1,000 rpm for 5 min, and the supernatant was discarded;
1.4. A complete culture medium was added to the cell pellet which was well mixed by gentle pipetting, the cell suspension was transferred to a culture flask, and the cells were cultured by supplementing culture medium.

Cell invasion assayed by Transwell

1. Test drugs were added according to different cell treatment groups: gemcitabine or 5-FU diluted in physiological saline only, or gemcitabine or 5-FU diluted in the sodium chloride deuterium oxide solution according to the concentrations shown in Table 3 (i.e. gemcitabine+deuterium oxide, or 5-FU+deuterium oxide), was separately used to treat A549 and HCT116 cells, and the cells were incubated for 24 hours in an incubator at 37°C, 5% $CO_2$;

2. After incubation of cells with the test drugs for 24 hours, serum was removed, and the cells were incubated under starvation with an incomplete culture medium for 24 hours;

3. Matrigel was placed at 4°C in advance to thaw overnight;

4. Thawed Matrigel was 1:1 diluted with the incomplete culture medium, 30 μl diluted Matrigel was added to the upper compartment of Transwell and incubated at 37°C for 120 min to gel the Matrigel;

5. Cells from different groups were digested, counted, and then adjusted to a density of $1 \times 10^5$ cells/ml with the incomplete culture medium, 100 μl cell suspension was added to the upper compartment (small compartment) of Transwell, and 500 μl medium containing 20% FBS was added to the lower compartment of Transwell;

6. A 24-well cell culture plate was placed in an incubator at 37°C and 5% $CO_2$ to culture the cells for 24 hours;

7. Cell counting: cells in the Matrigel and the upper compartment were wiped off with a cotton swab, the Transwell was removed, followed by inversion and air drying, 500 μl medium containing 0.1% crystal violet was added to the 24-well plate, the small compartment was placed in the medium to allow the membrane to be immersed in the dye, removed after holding at 37°C for 30 min, washed with PBS, and photographed in 3 fields along the diameter (200X magnification) for cell counting.

8. The assay was repeated one more time.

Results

Table 3. Inhibition of invasion of A549 and HCT-116 cells by deuterium oxide

| Group | Number of cells (HCT-116) | Number of cells (A549) |
|---|---|---|
| Control (Physiological saline) | 65.0±12.0 | 183.0±12.3 |
| Deuterium oxide (1.8%) | 29.3±5.1 | 74.3±11.0 |
| Deuterium oxide (3.7%) | 16.3±3.2 | 54.0±2.0 |
| Deuterium oxide (7.5%) | 3.7±0.6 | 43.0±4.4 |
| Deuterium oxide (15%) | 3.0±1.0 | 29.3±1.5 |
| Deuterium oxide (30%) | 1.70±0.6 | 22.0±1.7 |

In Table 3, for example, Deuterium oxide (1.8%) means that the concentration of deuterium oxide in the cell culture medium is 1.8% (v/v), and the same applies hereinafter.

The results demonstrate that deuterium oxide alone shows inhibition of invasion of A549 and HCT-116 cells, and the inhibition is proportional to its content.

Table 4. Inhibition of invasion of HCT-116 cells by 5-fluorouracil (5-FU)

| Group | Number of cells |
|---|---|
| Control | 76.0±4.6 |
| 5-FU (0.195ug/ml) | 66.0±2.6 |
| 5-FU (0.39ug/ml) | 47.3±4.0 |
| 5-FU (0.78ug/ml) | 31.3±1.5 |
| 5-FU (1.56ug/ml) | 23.0±4.6 |
| 5-FU (3.125ug/ml) | 12.0±2.0 |
| 5-FU (6.25ug/ml) | 10.7±1.5 |
| 5-FU (12.Sug/ml) | 6.3±0.6 |

In Table 4, for example, 5-FU (0.195 μg/ml) means that the concentration of 5-FU is 0.195 μg/ml in physiological saline as the medium.

Table 5. Inhibition of invasion of HCT-116 cells by 5-FU in combination with deuterium oxide

| Cell line | Group | Number of cells |
|---|---|---|
| HCT-116 | Control | 98.7±3.5 |
| | Deuterium oxide (30%)+5-FU (0.195ug/ml) | 8.7±1.5 |
| | Deuterium oxide (30%)+5-FU (0.39ug/ml) | 8.3±1.2 |
| | Deuterium oxide (30%)+5-FU (0.78ug/ml) | 6.3±0.6 |
| | Deuterium oxide (30%)+5-FU (1.56ug/ml) | 5.0±1.0 |
| | Deuterium oxide (30%)+5-FU (3.125ug/ml) | 5.7±0.6 |
| | Deuterium oxide (30%)+5-FU (6.25ug/ml) | 4.3±0.6 |
| | Deuterium oxide (30%)+5-FU (12.5ug/ml) | 3.3±0.6 |

In Table 5, for example, Deuterium oxide (30%)+5-FU (0.195 $\mu$g/ml) means that in the cell culture medium the concentration of deuterium oxide is 30% (v/v), and the concentration of 5-FU is 0.195 $\mu$g/ml.

The results demonstrate that the combination of 30% deuterium oxide and 5-FU significantly inhibited invasion of HCT-116 cells; in particular, when the concentration of 5-FU was only 0.195 $\mu$g/ml, the combination can significantly inhibit invasion of HCT-116 cells.

Table 6. Inhibition of invasion of A549 cells by gemcitabine (GEM)

| Group | Number of cells |
|---|---|
| Control | 178.3±13.8 |
| GEM (1.95nM) | 160.0±6.6 |
| GEM (3.9nM) | 112.7±3.5 |
| GEM (7.8nM) | 59.0±2.0 |
| GEM (15.6nM) | 51.7±3.1 |
| GEM (31.2nM) | 37.3±2.3 |
| GEM (62.5nM) | 22.3±0.6 |
| GEM (125nM) | 16.0±1.0 |
| GEM (250nM) | 9.7±2.1 |

Table 7. Inhibition of invasion of A549 cells by GEM in combination with deuterium oxide

| Cell line | Group | Number of cells |
|---|---|---|
| A549 | Control | 161.3±3.8 |
| | Deuterium oxide (30%)+GEM (1.95nM) | 31.0±1.0 |
| | Deuterium oxide (30%)+GEM (3.9nM) | 23.3±2.5 |
| | Deuterium oxide(30%)+GEM (7.8nM) | 21.0±1.0 |
| | Deuterium oxide (30%)+GEM (15.6nM) | 13.3±0.6 |
| | Deuterium oxide (30%)+GEM (31.2nM) | 10.3±0.6 |
| | Deuterium oxide (30%)+GEM (62.5nM) | 11.0±1.0 |
| | Deuterium oxide (30%)+GEM (125nM) | 8.0±1.0 |
| | Deuterium oxide (30%)+GEM (250nM) | 6.7±0.6 |

The results demonstrate that the combination of deuterium oxide and gemcitabine significantly inhibited invasion of A549 cells; in particular, when the concentration of gemcitabine was only 1.95 nM, the combination can significantly inhibit invasion of A549 cells.

The results demonstrate that deuterium oxide shows an inhibitory effect on metastasis of various tumor cells, proportional to its dose, and can be combined with other drugs to inhibit metastasis of tumor cells.

**Example 19**

Effect of deuterium oxide on the cell cycle of human lung cancer A549 cells and human colon cancer HCT-116 cells

Materials

Human lung cancer A549 cells and human colon cancer HCT-116 cells were provided by NANJING KEYGEN BIOTECH. CO., LTD., and cultured in an incubator at 37°C, 5% $CO_2$ and saturated humidity.

A KGA511 Cell cycle assay kit was provided by NANJING KEYGEN BIOTECH. CO., LTD.

The flow cytometer was FACS Calibur provided by Becton-Dickinson, US.

Test drugs: gemcitabine (GEM), 5-fluorouracil (5-FU), and the sodium chloride deuterium oxide solution prepared in Example 1.

Method:

Cell culturing

1. Cell resuscitation

1.1. Warm water at 37°C to 40°C was prepared, a cryogenic vial of cells was taken out of liquid nitrogen and immediately put into the 37°C-40°C warm water, followed by vigorous shaking until the cryogenic liquid was completely thawed;

1.2. The suspension of cryogenic cells was transferred to a centrifugal tube, 5 ml culture medium was added thereto, and the cells were well mixed in the medium by gentle pipetting;

1.3. The cell suspension was centrifuged at 800 to 1,000 rpm for 5 min, and the supernatant was discarded;

1.4. A complete culture medium was added to the cell pellet which was well mixed by gentle pipetting, the cell suspension was transferred to a culture flask, and the cells were cultured by supplementing culture medium.

2. Cell subculturing

2.1. The original culture medium was removed by pipetting when the cell coverage reached 80% to 90% in the culture bottle;

2.2. A suitable amount of trypsin (0.25%) was added to digest for 1 to 2 min;

2.3. When all the cells became round, an equal volume of culture medium containing serum was added to cease the digestion;

2.4. The cells were blown by pipetting to suspend, then drawn into a 15 ml centrifugal tube, and centrifuged at 1000 rpm for 5 min;

2.5. The supernatant was discarded, and the cells were re-suspended in 1 to 2 ml culture medium and transferred to a culture bottle for further culturing.

Detection of cell cycle by PI single staining

1. Cells growing in the exponential phase were digested and inoculated to a 6-well plate; on the next day, after the cells attached to the wall, cell culture media containing corresponding test drugs were added according to different groups, and a negative control group was also set;

2. After treatment with test drugs for 72 hours, cells were digested with 0.25% pancreatin (EDTA-free) and collected;

3. The cells were washed with PBS once (centrifuged at 2,000 rpm for 5 min), and $5 \times 10^5$ cells were collected;

4. The prepared suspension of individual cells was fixed with 70% (v/v) ethanol for 2 hours (or overnight), and stored at 4°C; before staining, the fixing solution was washed away with PBS (if necessary, the cell suspension was filtered once through a 200-mesh screen);

5. 100 μl RNase A was added, and the cells were water-bathed at 37°C for 30 min;

6. 400 μl PI was added and well mixed for staining, and the cells were kept at 4°C in darkness for 30 min;

7. In a detection apparatus, red fluorescence with an excitation wavelength of 488 nm was recorded.

Results

Table 8. Effect of deuterium oxide on the cell cycle of human colon cancer HCT-116 cells (%, $\bar{x} \pm S$ n=6)

| Group | G1 Phase | S Phase | G2 Phase |
|---|---|---|---|
| Control (physiological saline) | 82.53±1.76 | 9.40±1.83 | 8.06±1.89 |
| Deuterium oxide (30%, v/v) | 89.19±3.81 | 6.61±1.41 | 4.21±0.95 |
| 5-FU (12.5ug/ml in physiological saline) | 32.69±8.66 | 26.07±11.32 | 41.24±2.97 |
| Deuterium oxide (30%, v/v)+ 5-FU (12.5ug/ml) | 49.16±4.47 | 0.30±0.26 | 50.53±4.26 |

Table 9. Effect of deuterium oxide on the cell cycle of human lung cancer A549 cells (%, $\bar{x} \pm S$, n=6)

| Group | G1 Phase | S Phase | G2 Phase |
|---|---|---|---|
| Control (physiological saline) | 80.29±5.02 | 13.50±1.33 | 6.21±4.52 |
| Deuterium oxide (30%, v/v) | 86.23±3.69 | 0.31±0.28 | 13.47±3.94 |
| GM (250nM in physiological saline) | 73.73±3.73 | 15.55±5.51 | 10.71±2.39 |
| Deuterium oxide (30%, v/v)+ GM (250 nM) | 87.32±1.60 | 0.08±0.01 | 12.6±1.46 |

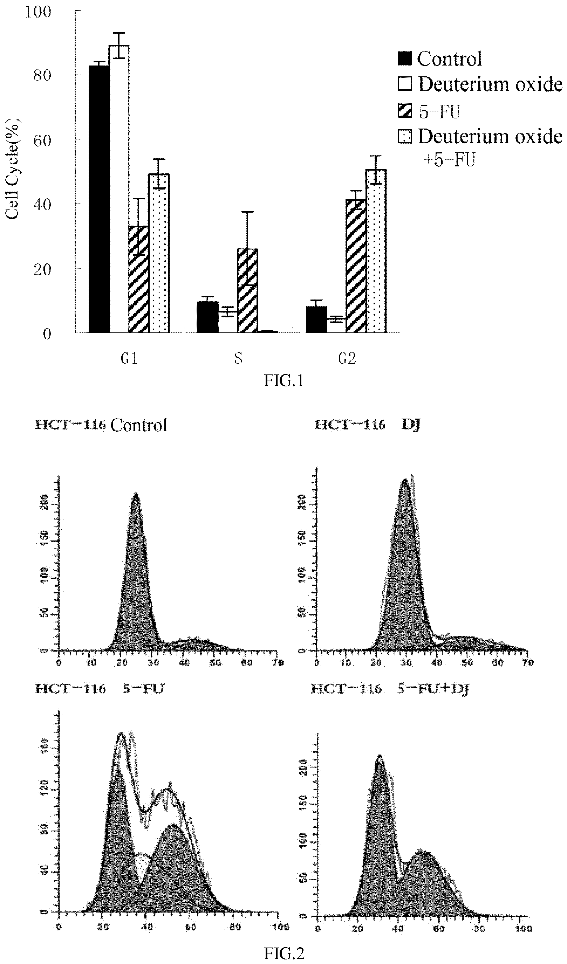

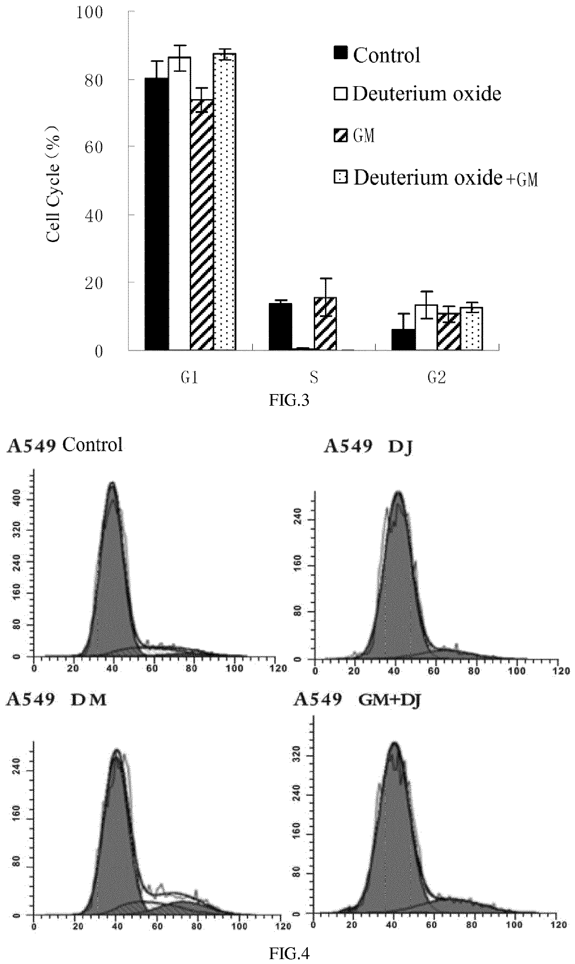

5-FU and gemcitabine are cytotoxic metabolite anti-tumor drugs, and mainly act on tumor cells in the DNA synthesis phase, i.e. the S phase. It can be seen in the results of Tables 8 and 9 and Figures 1-4 that deuterium oxide impeded tumor cells entering the G2 phase from the S phase; when deuterium oxide was combined with 5-FU and gemcitabine, 5-FU and gemcitabine killed the S-phase tumor cells more effectively, resulting in significant reduction in the S-phase tumor cells, and exerting a synergistic effect.

**Example 20**

Efficacy of intraperitoneal lavage with deuterium oxide in combination with 5-fluorouracil on Ehrlich ascites tumor (EAC)-bearing mice

Laboratory animal: 4 to 5 week-aged male ICR mice, provided by Shanghai SIPPR-BK Laboratory Animal Co. Ltd.

Cell lines: Ehrlich ascites tumor cells were provided by NANJING KEYGEN BIOTECH. CO., LTD.

Test drugs: 5-fluorouracil (5-FU), and the sodium chloride deuterium oxide solution prepared in Example 1.

Experimental method:

1. Modeling: EAC ascitic fluid was taken and adjusted to $1 \times 10^7$ cells/ml, which was inoculated into the peritoneal cavity of mice at 0.1 ml/mouse.

2. Grouping and dosing: the animals were randomized 3 days after inoculation, and meanwhile drugs were given to mice in each group, following the dosing scheme shown in Table 10.

Table 10. Grouping and dosing scheme.

| Groups | Drugs, I.P. administration | Inoculation | Dosing | Data |
|---|---|---|---|---|
| Group 1 | physiological saline 0.4 ml/animal | Giving NS for 3 days first, then giving drug on day 3, followed by inoculation | Then giving 0.4 ml NS on each day until death of the animal | |
| Group 2 | 5-FU 20 mg/kg alone (in physiological saline) | Giving 5-FU for 3 days first, then giving drug on day 3, followed by inoculation | Then giving 5-FU 20 mg/kg on each day for 7 successive days (10 days in total), then discontinuing the drug | Days that the animal had survived were recorded |
| Group 3 | 5-FU 30 mg/kg alone (in physiological saline) | Giving 5-FU for 3 days first, then giving drug on day 3, followed by inoculation | Then giving 5-FU 30 mg/kg on each day for 7 successive days (10 days in total), then discontinuing the drug | Days that the animal had survived were recorded |
| Group 4 | NaCl deuterium oxide solution, alone, 0.4 ml/animal | Giving NaCl deuterium oxide solution for 3 days first, then giving drug on day 3, followed by inoculation | Then giving 0.4 ml NaCl deuterium oxide solution on each day until death of animal, without drug discontinuance | Days that the animal had survived were recorded, |
| **The above 4 groups and Group 5, Group 6, Group 7 below, seven groups in total:** | | | | |
| Group 5 | NaCl deuterium oxide solution 0.1 ml/animal+ 5-FU 20mg/kg | Giving drug for 3 days first, then giving drug on day 3, followed by inoculation | Then giving drug on each day for 10 days in total, then discontinuing the drug | Days that the animal had survived were recorded |
| Group 6 | NaCl deuterium oxide solution 0.2 ml/animal+ 5-FU 20mg/kg | Giving drug for 3 days first, then giving drug on day 3, followed by inoculation | Then giving drug on each day for 10 days in total, then discontinuing the drug | Days that the animal had survived were recorded |
| Group 7 | NaCl deuterium oxide solution 0.4 ml/animal+ 5-FU 20mg/kg | Giving drug for 3 days first, then giving drug on day 3, followed by inoculation | Then giving drug on each day for 10 days in total, then discontinuing the drug | Days that the animal had survived were recorded |
| | | | | |
| **For the 4 groups below, NaCl deuterium oxide solution (0.6 ml/animal) was given 3 times by intraperitoneal lavage followed by intraperitoneal administration of drugs to corresponding groups** | | | | |
| Group 8 | physiological saline 0.4 ml/animal | Giving NS for 3 days first, then giving drug on day 3, followed by inoculation | Then giving 0.4 ml NS on each day until death of the animal | Days that the animal had survived were recorded |

(continued)

| For the 4 groups below, NaCl deuterium oxide solution (0.6 ml/animal) was given 3 times by intraperitoneal lavage followed by intraperitoneal administration of drugs to corresponding groups | | | | |
|---|---|---|---|---|
| Group 9 | NaCl deuterium oxide solution 0.1 ml/animal+ 5-FU, 20mg/kg | Giving drug for 3 days first, then giving drug on day 3, followed by inoculation | Then giving drug on each day for 10 days in total, then discontinuing the drug | Days that the animal had survived were recorded |
| Group 10 | NaCl deuterium oxide solution 0.2 ml/animal+ 5-FU, 20mg/kg | Giving drug for 3 days first, then giving drug on day 3, followed by inoculation | Then giving drug on each day for 10 days in total, then discontinuing the drug | Days that the animal had survived were recorded |
| Group 11 | NaCl deuterium oxide solution 0.4 ml/animal + 5-FU, 20mg/kg | Giving drug for 3 days first, then giving drug on day 3, followed by inoculation | Then giving drug on each day for 10 days in total, then discontinuing the drug | Days that the animal had survived were recorded |

In Table 10, "Sodium chloride deuterium oxide solution 0.1 ml/animal + 5-FU 20 mg/kg" means that each mouse was given 0.1 ml sodium chloride deuterium oxide solution, in which 5-FU had been dissolved to make a dose of 20 mg/kg.

3. Observation of indicators

After the dosing, ascitic fluid was taken from 3 mice from each group, the volume of the ascitic fluid was measured, the number of tumor cells in the ascitic fluid was counted, and the rest of animals were continuously fed. Median survival time (MST) was recorded for animals in each group to evaluate the survival time for each group.
The comparison between the treatment group and the control group was expressed by T/C (%) which is calculated by the following equation:

$$T/C \% = \frac{T\,MST}{C\,MST} \times 100\%$$

T MST: MST of the treatment group; C MST: MST of the negative control group.
The evaluation criterion uses 125% as a cut-off threshold. When T/C%$\geq$125%, effective, otherwise ineffective.

4. Statistics

The mean value was expressed in X$\pm$SD. Inter-group analysis was statistically performed with t test. The results were statistically analyzed using SPSS (Statistical Package for the Social Science) 17.0.

5. Results

5.1. Survival time: effect of intraperitoneal administration of drugs on survival time (in days) of animals

Table 11. Effect of intraperitoneal administration of drugs on survival time (in days) of animals ($\bar{x}\pm$SD, n=8)

| Groups | MST (days) | T/C (%) |
|---|---|---|
| Physiological saline (0.4 ml/animal) | 14.1$\pm$1.9 | |
| 5-FU (20mg/kg) (in physiological saline) | 21.8$\pm$2.9* | 154.6% |
| 5-FU (30mg/kg) (in physiological saline) | 22.3$\pm$4.9* | 158.1% |
| NaCl deuterium oxide solution (0.4ml/animal) | 17.2$\pm$3.2** | 121.9% |

(continued)

| Groups | MST (days) | T/C (%) |
|---|---|---|
| NaCl deuterium oxide solution (0.1 ml/animal)+ 5-FU (20mg/kg) | 21.4±3.1* | 151.7% |
| NaCl deuterium oxide solution (0.2 ml/animal)+ 5-FU (20mg/kg) | 24.1±2.7* | 170.9% |
| NaCl deuterium oxide solution (0.4 ml/animal)+ 5-FU (20mg/kg) | 25.7±2.6* | 182.2% |
| Compared to the negative control group: * P<0.001, ** P<0.05. | | |

Table 12. Effect of intraperitoneal lavage with sodium chloride deuterium oxide solution, followed by intraperitoneal administration of drugs, on survival time (in days) of animals ($\bar{x}\pm$SD, n=8)

| Groups | MST (days) | T/C (%) |
|---|---|---|
| Physiological saline (0.4 ml/animal) | 15.3±1.9 | |
| NaCl deuterium oxide solution (0.1ml/ animal) + 5-FU (20mg/kg) | 26.1±3.7* | 170.5% |
| NaCl deuterium oxide solution (0.2ml/ animal) + 5-FU (20mg/kg) | 29.1±3.1* | 190.1 % |
| NaCl deuterium oxide solution (0.4ml/ animal) + 5-FU (20mg/kg) | 34.5±3.3* | 225.4% |
| Compared to the negative control group: * P<0.001. | | |

The results demonstrate that, as compared to the physiological saline control group, the group receiving combined sodium chloride deuterium oxide solution and 5-FU showed an extended MST, with a P value <0.01 and T/C(%)>150%, which was also better than the group receiving 5-FU alone.

Intraperitoneal lavage with the sodium chloride deuterium oxide solution first, followed by intraperitoneal administration of drugs, can achieve a significantly extended MST in mice, longer than the MST resulting from only intraperitoneal administration of drugs in the sodium chloride deuterium oxide solution, namely 29.1±3.1 days vs. 24.1±2.7 days; and 34.5±3.3 days vs. 25.7±2.6 days, both having a P value<0.05, demonstrating an advantage of intraperitoneal lavage with deuterium oxide followed by administration of drugs.

5.2 Ascitic fluid: after administration of drugs, 3 mice from each group were sacrificed by cervical dislocation and disinfected by a routine procedure; ascitic fluid was collected by opening the abdomen under sterile conditions, and the volume thereof was measured.

Table 13. Effect of intraperitoneal administration of drugs in sodium chloride deuterium oxide solution on the volume of ascitic fluid ($\bar{x}\pm$SD, n=3)

| Groups | Ascitic fluid (ml) |
|---|---|
| Physiological saline (0.4 ml/animal) | 14.3±1.4 |
| 5-FU (20mg/kg)(in physiological saline) | 6.9±0.9* |
| 5-FU (30mg/kg)(in physiological saline) | 4.9±0.5* |
| NaCl deuterium oxide solution (0.4 ml/animal) | 9.9±0.3** |
| NaCl deuterium oxide solution (0.1 ml/animal)+ 5-FU (20mg/kg) | 7.0±1.3* |
| NaCl deuterium oxide solution (0.2 ml/animal)+ 5-FU (20mg/kg) | 5.9±0.5* |

(continued)

|  | Groups | Ascitic fluid (ml) |
|---|---|---|
|  | NaCl deuterium oxide solution (0.4 ml/animal)+ 5-FU (20mg/kg) | 4.7±0.3* |
|  | Compared to the negative control group: * P<0.001, ** P<0.05. | |

Table 14. Effect of intraperitoneal lavage with sodium chloride deuterium oxide solution first, followed by intraperitoneal administration of drugs, on the volume of ascitic fluid ($\bar{x}$±SD, n=3)

| Groups | Ascitic fluid (ml) |
|---|---|
| Physiological saline (0.4 ml/animal) | 12.2±0.7 |
| NaCl deuterium oxide solution (0.1ml/animal) + 5-FU (20mg/kg) | 5.8±0.7* |
| NaCl deuterium oxide solution (0.2ml/animal) + 5-FU (20mg/kg) | 3.3±0.1* |
| NaCl deuterium oxide solution (0.4ml/animal) + 5-FU (20mg/kg) | 2.6±0.1 * |
| Compared to the negative control group: * P<0.001. | |

The results demonstrate that, as compared to the physiological saline control group, the group receiving the sodium chloride deuterium oxide solution showed a reduced volume of ascitic fluid with P<0.05; as compared to the group receiving 5-FU alone, the group receiving the sodium chloride deuterium oxide solution in combination with 5-FU also showed a reduced volume of ascitic fluid, namely 7.0±1.3 ml, 5.9±0.5 ml, 4.7±0.3 ml vs 6.9±0.9 ml, all with P<0.05.

Groups upon intraperitoneal lavage with the sodium chloride deuterium oxide solution, followed by intraperitoneal administration of drugs, showed a significantly reduced volume of ascitic fluid as compared to the groups receiving only intraperitoneal administration of drugs without lavage, namely 5.8±0.7 ml vs.7.0±1.3 ml; 3.3±0.1 ml vs. 5.9±0.5 ml; 2.6±0.1 ml vs. 4.7±0.3 ml, all with P<0.01, demonstrating an advantage of intraperitoneal lavage with deuterium oxide followed by administration of drugs.

**Example 21**

Inhibition of liver cancer H22 ascites tumor in tumor-bearing mice by (42°C) hyperthermic intraperitoneal retention instillation using deuterium oxide in combination with cisplatin and using deuterium oxide in combination with cisplatin and epirubicin

Experimental method:

Animal: 4 to 5 week-aged male Kunming mice, provided by Shanghai SIPPR-BK Laboratory Animal Co. Ltd.

Cell lines: Liver cancer H22 ascites tumor cell line was provided by NANJING KEYGEN BIOTECH. CO., LTD.

Modeling: 0.2 ml suspension of H22 tumor cells containing $1\times10^7$ cells/ml ($2\times10^6$ H22 cells in total) was inoculated into the peritoneal cavity of mice.

Test drugs: cisplatin, epirubicin, and the Ringer's deuterium oxide solution as prepared in Example 5.

Grouping and dosing: the mouse model was established 7 to 8 days after inoculation, and the mice were randomized with 18 animals/group.

Cisplatin and epirubicin were dissolved in a 42°C Ringer's deuterium oxide solution, and used to perfuse the peritoneal cavity of the tumor-bearing mice.

1). Blank control group (42°C normal Ringer's solution for injection, 10 ml/kg);

2). Normal chemo group (cisplatin 0.6 mg/kg + 42°C normal Ringer's solution for injection, 10 ml/kg);

3). Low-dose deuterium oxide + cisplatin group (42°C Ringer's deuterium oxide solution, 5 ml/kg + cisplatin 0.6 mg/kg);

4). Mid-dose deuterium oxide + cisplatin group (42°C Ringer's deuterium oxide solution, 20 ml/kg + cisplatin 0.6 mg/kg);

5). High-dose deuterium oxide + cisplatin group (42°C Ringer's deuterium oxide solution, 40 ml/kg + cisplatin 0.6 mg/kg);

6). Low-dose deuterium oxide + cisplatin + epirubicin group (42°C Ringer's deuterium oxide solution, 5 ml/kg + cisplatin 0.6 mg/kg + epirubicin 0.2 mg/kg);

7). Mid-dose deuterium oxide + cisplatin + epirubicin group (42°C Ringer's deuterium oxide solution, 20ml/kg + cisplatin 0.6 mg/kg + epirubicin 0.2 mg/kg);

8). High-dose deuterium oxide + cisplatin + epirubicin group (42°C Ringer's deuterium oxide solution, 40ml/kg + cisplatin 0.6 mg/kg + epirubicin 0.2 mg/kg);

The peritoneal cavity of mice was delivered with the above compositions which were retained for 10 min in the peritoneal cavity after being introduced, which was repeated for 3 times. The instillation was performed once another day for 5 successive times. The body weight and abdomen circumference of mice were measured on each day before administration of the drugs, and the daily living status of mice was observed. 24 hours after the administration of drugs was completed (on day 11), 8 mice from each group were sacrificed, the volume of ascitic fluid was measured, and bodies of the mice were dissected to observe the peritoneal organs and metastasis to lung. The rest of mice were observed for their survival time, and the extension of life was calculated.

4. Observation of indicators: the same as Example 21.

5. Statistics: the same as Example 21.

6. Results

Table 15. Effect of 42°C intraperitoneal instillation with deuterium oxide in combination with cisplatin and epirubicin on survival time (in days) of animals ($\bar{x}\pm$SD, n=10)

| Groups | MST(days) | T/C(%) |
|---|---|---|
| Blank control group (Ringer's solution for injection, 5 ml/kg) | 13.2±1.9 | |
| Normal chemo group (cisplatin 0.6 mg/kg + Ringer's solution for injection 5ml/kg) | 18.1±3.2* | 137.1 |
| Low-dose deuterium oxide+cisplatin group (Ringer's deuterium oxide solution 5ml/kg+cisplatin 0.6mg/kg) | 20.3±2.2* | 153.7 |
| Mid-dose deuterium oxide+cisplatin group (Ringer's deuterium oxide solution 20ml/kg+cisplatin 0.6mg/kg) | 24.7±1.8* | 187.1 |
| High-dose deuterium oxide+cisplatin group (Ringer's deuterium oxide solution 40ml/kg+cisplatin 0.6mg/kg) | 26.1±5.9* | 197.7 |
| Low-dose deuterium oxide+cisplatin group +epirubicin (Ringer's deuterium oxide solution 5ml/kg+cisplatin 0.6mg/kg+epirubicin 0.2mg/kg) | 24.3±3.3* | 184.0 |
| Mid-dose deuterium oxide+cisplatin group +epirubicin (Ringer's deuterium oxide solution 20ml/kg+cisplatin 0.6mg/kg+epirubicin 0.2mg/kg) | 28.6±6.4* | 216.6 |
| High-dose deuterium oxide+cisplatin group +epirubicin (Ringer's deuterium oxide solution 40ml/kg+cisplatin 0.6mg/kg+epirubicin 0.2mg/kg) | 32.1±3.1* | 243.1 |
| Compared to the negative control group: * $P<0.001$. | | |

Table 16. Effect of 42°C intraperitoneal instillation with deuterium oxide in combination with cisplatin and epirubicin on the volume of ascitic fluid ($\bar{x}\pm$SD n=8)

| Groups | Ascitic fluid (ml) |
|---|---|
| Blank control group (Ringer's solution for injection, 5ml/kg) | 14.85±2.56 |

(continued)

| Groups | Ascitic fluid (ml) |
|---|---|
| Normal chemo group (cisplatin 0.6mg/kg + Ringer's solution for injection 5 ml/kg) | 7.30±2.32* |
| Low-dose deuterium oxide+cisplatin group (Ringer's deuterium oxide solution 5ml/kg+cisplatin 0.6mg/kg) | 6.55±3.95* |
| Mid-dose deuterium oxide+cisplatin group (Ringer's deuterium oxide solution 20ml/kg+cisplatin 0.6mg/kg) | 4.93±3.05* |
| High-dose deuterium oxide+cisplatin group (Ringer's deuterium oxide solution 40ml/kg+cisplatin 0.6mg/kg) | 4.08±1.22* |
| Low-dose deuterium oxide+cisplatin group +epirubicin (Ringer's deuterium oxide solution 5ml/kg+cisplatin 0.6mg/kg+epirubicin 0.2mg/kg); | 3.11±0.56** |
| Mid-dose deuterium oxide+cisplatin group +epirubicin (Ringer's deuterium oxide solution 20ml/kg+cisplatin 0.6mg/kg+epirubicin 0.2mg/kg); | 2.49±0.62** |
| High-dose deuterium oxide+cisplatin group +epirubicin (Ringer's deuterium oxide solution 40ml/kg+cisplatin 0.6mg/kg+epirubicin 0.2mg/kg). | 0.44±0.46** |
| Compared to the negative control group: * P<0.001, ** P<0.0001. | |

Cisplatin is a representative anti-tumor platinum preparation, epirubicin is a broad-spectrum tumor antibiotic, and deuterium oxide has a broad-spectrum effect of enhancing anti-tumor efficacy. The results demonstrate that the hyperthermic (42°C) intraperitoneal instillation with deuterium oxide in combination with cisplatin and epirubicin can significantly extend the survival time of mice bearing H22 ascites tumors, inhibits production of ascitic fluid in mice bearing H22 ascites tumors, and improves quality of life of the tumor-bearing mice, thereby exhibiting an anti-tumor effect.

**Example 22**

Inhibition of sarcoma S180 (ascites type) in tumor-bearing mice by (42°C) hyperthermic intraperitoneal retention instillation using deuterium oxide in combination with oxaliplatin and using deuterium oxide in combination with oxaliplatin and mitomycin

Experimental method:

Animal: 4 to 5 week-aged male Kunming mice, provided by Shanghai SIPPR-BK Laboratory Animal Co. Ltd.
Cell lines: sarcoma S180 (ascites type) cell line was provided by NANJING KEYGEN BIOTECH. CO., LTD.
Modeling: 0.2 ml suspension of sarcoma S180 cells containing $1×10^7$ cells/ml was inoculated into the peritoneal cavity of mice.
Test drugs: oxaliplatin, mitomycin, and the glucose deuterium oxide solution as prepared in Example 2.
Grouping and dosing: the mouse model was established 7 to 8 days after inoculation, and the mice were randomized with 12 animals/group.
Oxaliplatin and mitomycin were dissolved in a 42°C glucose deuterium oxide solution, and used to perfuse the peritoneal cavity of the tumor-bearing mice.

1). Blank control group (42°C normal glucose injection, 5 ml/kg);
2). Normal chemo group (oxaliplatin 0.8 mg/kg + 42°C normal glucose injection, 5 ml/kg);
3). Low-dose deuterium oxide + oxaliplatin group (42°C glucose deuterium oxide solution, 5 ml/kg + oxaliplatin 0.8 mg/kg);
4). Mid-dose deuterium oxide + oxaliplatin group (42°C glucose deuterium oxide solution, 20 ml/kg + oxaliplatin 0.8 mg/kg);
5). High-dose deuterium oxide + oxaliplatin group (42°C glucose deuterium oxide solution, 40 ml/kg + oxaliplatin 0.8 mg/kg);
6). Low-dose deuterium oxide + oxaliplatin + mitomycin group (42°C glucose deuterium oxide solution, 5 ml/kg + oxaliplatin 0.8 mg/kg + mitomycin 0.4 mg/kg);
7). Mid-dose deuterium oxide + oxaliplatin + mitomycin group (42°C glucose deuterium oxide solution, 20 ml/kg + oxaliplatin 0.8 mg/kg + mitomycin 0.4 mg/kg);

8). High-dose deuterium oxide + oxaliplatin + mitomycin group (42°C glucose deuterium oxide solution, 40 ml/kg + oxaliplatin 0.8 mg/kg + mitomycin 0.4 mg/kg).

The peritoneal cavity was perfused with the 42°C glucose deuterium oxide solution in combination with the above compositions, which were retained for 10 min in the peritoneal cavity after being introduced, which was repeated for 3 times. The instillation was performed once another day for 5 successive times. The body weight and abdomen circumference of mice were measured on each day before administration of the drugs, and the daily living status of mice was observed. 24 hours after the administration of drugs was completed (on day 11), 4 mice from each group were sacrificed, the volume of ascitic fluid was measured, and bodies of the mice were dissected to observe the peritoneal organs and metastasis to lung. The rest of mice were observed for their survival time, and the extension of life was calculated.

4. Observation of indicators: the same as Example 21.

5. Statistics: the same as Example 21.

6. Results

Table 17. Effect of 42°C intraperitoneal instillation with deuterium oxide in combination with oxaliplatin and mitomycin on survival time (in days) of animals ($\bar{x} \pm SD$, n=8)

| Group | MST(days) | T/C(%) |
|---|---|---|
| Blank control group (glucose injection, 5 ml/kg) | 12.2±3.0 | |
| Normal chemo group (oxaliplatin0.8mg/kg +glucose injection 5ml/kg) | 21.7±2.8* | 177.6 |
| Low-dose deuterium oxide+oxaliplatin (glucose deuterium oxide solution 5ml/kg+oxaliplatin 0.8mg/kg) | 23.4±3.2* | 191.8 |
| Mid-dose deuterium oxide+oxaliplatin (glucose deuterium oxide solution 20ml/kg+oxaliplatin 0.8mg/kg) | 27.7±4.9* | 227.0 |
| High-dose deuterium oxide+oxaliplatin (glucose deuterium oxide solution 40ml/kg+oxaliplatin 0.8mg/kg) | 31.1±6.1* | 254.9 |
| Low-dose deuterium oxide+oxaliplatin+mitomycin (glucose deuterium oxide solution 5ml/kg+oxaliplatin 0.8mg/kg+mitomycin 0.4 mg/kg) | 26.4 | 216.3 |
| Mid-dose deuterium oxide+oxaliplatin+mitomycin (glucose deuterium oxide solution 20ml/kg+oxaliplatin 0.8mg/kg+mitomycin 0.4 mg/kg ) | 30.1 | 246.7 |
| High-dose deuterium oxide+oxaliplatin+mitomycin (glucose deuterium oxide solution 40ml/kg+oxaliplatin 0.8mg/kg+mitomycin 0.4 mg/kg ) | 32.8 | 268.8 |
| Compared to the negative control group: * P<0.0001. | | |

Table 18. Effect of 42°C intraperitoneal instillation with deuterium oxide in combination with oxaliplatin and mitomycin on the volume of ascitic fluid ($\bar{x} \pm SD$, n=8)

| Group | Ascitic fluid (ml) |
|---|---|
| Blank control group (glucose injection , 5 ml/kg) | 16.5±4.3 |
| Normal chemo group (oxaliplatin0.8mg/kg +glucose injection 5ml/kg) | 8.7±1.9* |
| Low-dose deuterium oxide +oxaliplatin (oxaliplatin 0.8mg/kg +glucose deuterium oxide solution 5ml/kg) | 8.1±2.8* |

(continued)

| Group | Ascitic fluid (ml) |
|---|---|
| Mid-dose deuterium oxide +oxaliplatin (oxaliplatin 0.8mg/kg +glucose deuterium oxide solution 20ml/kg) | 7.3±2.9* |
| High-dose deuterium oxide +oxaliplatin (oxaliplatin 0.8mg/kg +glucose deuterium oxide solution 40ml/kg) | 2.1±1.3** |
| Low-dose deuterium oxide+oxaliplatin+mitomycin (oxaliplatin 0.8mg/kg+mitomycin 0.4 mg/kg+glucose deuterium oxide solution 5ml/kg) | 7.5±3.1* |
| Mid-dose deuterium oxide+oxaliplatin+mitomycin (oxaliplatin 0.8mg/kg+mitomycin 0.4 mg/kg +glucose deuterium oxide solution 20ml/kg) | 3.7±1.6** |
| High-dose deuterium oxide+oxaliplatin+mitomycin (oxaliplatin 0.8mg/kg+mitomycin 0.4 mg/kg +glucose deuterium oxide solution 40ml/kg) | 1.1±0.5** |
| Compared to the negative control group: * P<0.0001, ** P<0.00001. | |

Oxaliplatin is a third-generation anti-tumor platinum preparation, mitomycin is an anti-tumor antibiotic to which tumor cells in G0 to S phases are sensitive, and deuterium oxide has a broad-spectrum effect of enhancing anti-tumor efficacy, to which tumor cells in the S phase are also sensitive. Their combination can further enhance anti-tumor efficacy. The results demonstrate that the hyperthermic (42°C) intraperitoneal instillation with deuterium oxide in combination with oxaliplatin and mitomycin can also extend the survival time of mice bearing sarcoma S180, and inhibits production of ascitic fluid in mice bearing sarcoma S180. Examples 21 and 22 indicate that combination of deuterium oxide with platinum preparations and anti-tumor antibiotics has general applicability in inhibition of growth of various tumors.

**Example 23**

Inhibition of growth of xenograft tumors from human colon cancer HCT-166 cells in nude mice by intravenous injection of deuterium oxide in combination with 5-fluorouracil

Animal: 6 to 7 week-aged female BALB/c nude mice, each weighing 18 to 20 g, provided by Shanghai SLAC Laboratory Animal Co. Ltd.

Cell lines: human colon cancer cells HCT-116 were provided by NANJING KEYGEN BIOTECH. CO., LTD.

Test drugs: 5-fluorouracil (5-FU), and the glucose (5%) deuterium oxide solution (containing 5 g glucose per 100 ml) prepared in Example 2.

Experimental method:

1. Modeling

Tumor cells were resuscitated and cultured according to a routine procedure, a suspension of cultured HCT-116 cells was collected, sterile physiological saline was added thereto to make a suspension at a concentration of $1 \times 10^7$ cells/ml, and 0.1 ml of the suspension was subcutaneously inoculated into the right axillary fossa of each nude mouse.

2. Grouping and dosing scheme

The diameter of xenograft tumors in the nude mice was measured with a vernier caliper, and 10 days after inoculation, when tumors grew to 100 to 110 cm$^3$, the animals were randomized with 8 mice per group. Meanwhile drugs were administered by injection into tail vein, once every three days, for 21 successive days. By measuring the tumor diameter, the anti-tumor efficacy of test samples was dynamically observed, and the body weight of animals was also recorded to observe the toxicity of the test samples. 21 days after administration, the nude mice were sacrificed, and tumors were harvested by surgery, weighed and photographed. Bone marrow nucleated cells were isolated by a routine method, DNA was extracted from the cells, the OD value was measured in a UV-260 spectrometer as a representative of the DNA content, and inhibition of DNA synthesis by the test samples was observed.

The test used 7 groups in total, with 8 animals per group.

1) Negative control group: 5% glucose injection, 10 ml/kg;
2) Paclitaxel positive control group: paclitaxel diluted in physiological saline (8 mg/kg);

3) High-dose 5-FU positive control group: 5-FU diluted in 5% glucose injection (30 mg/kg);
4) Low-dose 5-FU positive control group: 5-FU diluted in 5% glucose injection (12 mg/kg).
5) Test group 1: 5-FU (12 mg/kg) diluted in the glucose deuterium oxide solution (5 ml/kg).
6) Test group 1: 5-FU (12 mg/kg) diluted in the glucose deuterium oxide solution (10 ml/kg).
7) Test group 1: 5-FU (12 mg/kg) diluted in the glucose deuterium oxide solution (20 ml/kg).

3. Observation of indicators

1) Anti-tumor activity evaluation by relative tumor volume

Tumor volume (TV) was calculated by the following equation:

$$TV = 1/2 \times a \times b^2,$$

wherein a and b refer to length and width, respectively.

From the measurements, relative tumor volume (RTV) was calculated by the following equation:

$$RTV = V_t/V_0$$

wherein $V_0$ is the tumor volume measured on initial administration (i.e. $d_0$), and Vt is the tumor volume measured each time afterwards.

Relative tumor growth ratio T/C(%) was calculated by the following equation:

$$T/C(\%) = \frac{T_{RTV}}{C_{RTV}} \times 100$$

$T_{RTV}$: RTV of the treatment group; $C_{RTV}$: RTV of the negative control group.

Criteria of efficacy evaluation: T/C%>40%, not efficacious; T/C%≤40% and statistically P<0.05, efficacious.

2) Anti-tumor activity evaluation by tumor weight

Inhibition of tumor growth (%) was calculated by the following equation:

$$\text{Inhibition of tumor growth} = \frac{ATW_{NCG} - ATW_{DG}}{ATW_{NCG}} \times 100\%$$

$ATW_{NCG}$: Average tumor weight in negative control group
$ATW_{DG}$: Average tumor weight in drug-dosed group

Criteria of efficacy evaluation: Inhibition of tumor growth<40%, not efficacious; Inhibition of tumor growth≥40% and statistically P<0.05, efficacious.

3) Observation of drug's inhibition of bone marrow and monitoring of drug's toxicity based on bone marrow DNA content.

4. Statistics

The mean value was expressed in X±SD. Inter-group analysis was statistically performed with t test. The results were statistically analyzed using SPSS (Statistical Package for the Social Science) 17.0.

5. Results

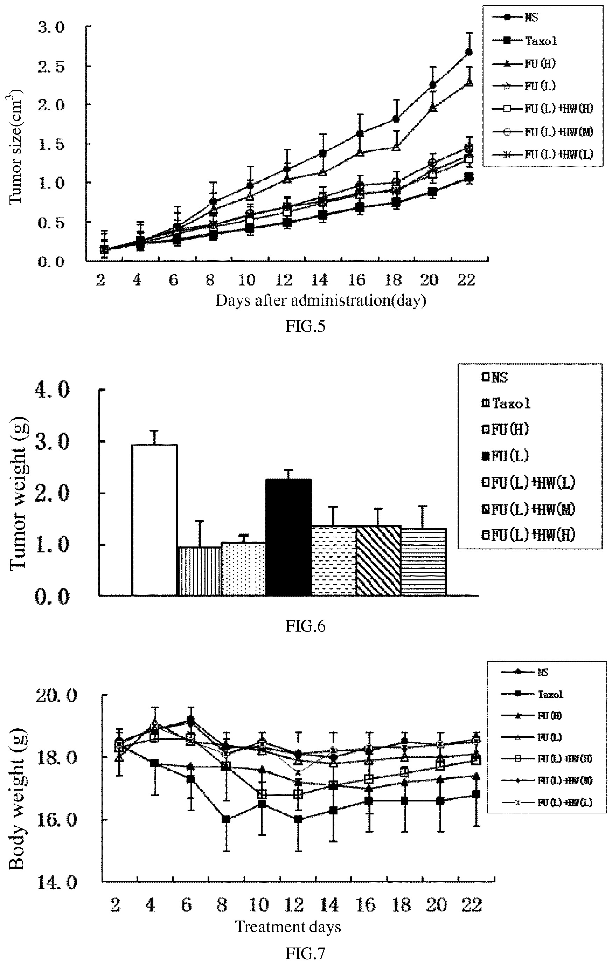

**Anti-tumor activity evaluation by xenograft tumor volume:** the results demonstrate that deuterium oxide in combination with low-dose 5-FU showed a good inhibitory effect on the xenograft tumors; 21 days after administration, the tumor volumes were $1.22\pm0.15$ cm$^3$ in the group receiving the glucose deuterium oxide solution (5 mg/kg) in combination with 5-FU, $1.45\pm0.25$ cm$^3$ in the group receiving the glucose deuterium oxide solution (10 mg/kg) in combination with 5-FU, and $1.27\pm0.24$ cm$^3$ in the group receiving the glucose deuterium oxide solution (20 mg/kg) in combination with 5-FU, all with a T/C<40% as compared to the negative control group. The group receiving low-dose 5-FU alone showed a reduced tumor volume to $2.27\pm0.30$ cm$^3$ with a T/C of 80.91%, which is greater than 40% and does not meet the criteria for pharmacological efficacy. Therefore, when combined with 5-FU, deuterium oxide significantly enhances the anti-tumor efficacy of 5-FU, as shown in Figure 5.

**Anti-tumor activity evaluation by xenograft tumor weight:** the results demonstrate that deuterium oxide in combination with low-dose 5-FU showed a good inhibitory effect on the xenograft tumors; 21 days after administration, the inhibitions were 56.32% in the group receiving the glucose deuterium oxide solution (5 mg/kg) in combination with 5-FU, 53.70% in the group receiving the glucose deuterium oxide solution (10 mg/kg) in combination with 5-FU, and 54.22% in the group receiving the glucose deuterium oxide solution (20 mg/kg) in combination with 5-FU, all with a T/C>40% and P<0.001 as compared to the tumor weight in the negative control group, and all meeting the criteria for pharmacological efficacy. The group receiving low-dose 5-FU alone showed an inhibition of 23.64% and does not meet the criteria for pharmacological efficacy. Therefore, when combined with 5-FU, deuterium oxide significantly enhances the anti-tumor efficacy of 5-FU, as shown in Table 19 and Figure 6.

Table 19. Effect of deuterium oxide in combination with 5-FU on xenograft tumors from human colon cancer HCT-116 cells in nude mice ($\bar{x}\pm$SD n=8)

| Group | Dose $\times$ Times | Body weight (g) (Initial/End) | Tumor weight (g) | Inhibition (%) | Bone marrow DNA content (OD) |
|---|---|---|---|---|---|
| Negative Control NS | 10 ml/kg | 18.5/+0.4 | $2.92\pm0.27$ | -- | $0.950\pm0.2711$ |
| Paclitaxel positive control Taxol | 8mg/kg$\times$11 | 18.4/-1.2 | $0.94\pm0.25$ | 67.80%* | $0.841\pm0.3014$** |
| 5-FU positive control 5-FU(H) | 30 mg/kg $\times$ 11 | 18.4/-1.0 | $1.04\pm0.13$ | 64.38%* | $0.836\pm0.5023$** |
| 5-FU low-dose 5-FU(L) | 12 mg/kg $\times$ 11 | 18.0/-0.6 | $2.23\pm0.2$ | 23.63% | $0.897\pm0.3783$** |
| Test group 1 5-FU(L)+HW(L) | Glucose deuterium oxide solution (5 ml/kg) + 5-FU(12 mg/kg)$\times$ 11 | 18.3/-0.4 | $1.34\pm0.38$ | 54.10%* | $0.913\pm0.5122$ |
| Test group 2 5-FU(L)+HW(M) | Glucose deuterium oxide solution (10 ml/kg) + 5-FU(12 mg/kg)$\times$ 11 | 18.5/+0.2 | $1.35\pm0.34$ | 53.76%* | $0.901+0.4655$ |
| Test group 3 5-FU(L)+HW(H) | Glucose deuterium oxide solution (20 ml/kg) + 5-FU(12 mg/kg)$\times$ 11 | 18.4/+0.2 | $1.28\pm0.46$ | 56.16%* | $0.921+0.2910$ |
| Compared to the negative control group: * P<0.001, ** P<0.05. Inhibition of tumor growth (%) > 40% indicates pharmacological efficacy. | | | | | |

**Toxicity reduction:** importantly, in the high-dose paclitaxel and 5-FU positive control groups, despite apparent inhibition of tumor growth, the body weight of tumor-bearing mice dropped greatly from $18.4\pm0.5$ g to $16.8\pm0.9$ g and to $17.4\pm0.5$ g, respectively, and the bone marrow DNA content also decreased, indicating significant toxicity of paclitaxel and 5-FU to animals. The glucose deuterium oxide solution in combination with 5-FU (10 mg/kg, one third of that of control) can achieve the same efficacy as high-dose paclitaxel and 5-FU (30 mg/kg) (equivalence), while the body weight of the tumor-bearing mice during dosing did not drop, and the bone marrow DNA content did not decrease, without the toxicity caused by high-dose paclitaxel and 5-FU. Because toxicity of anti-tumor drugs is very harmful to patients and even unacceptable to some weak or aged patients, chemotherapeutic drugs having low toxic side effects and a strong killing effect are urgently needed in tumor treatment. Hence, deuterium oxide in combination with low-dose 5-FU has a great advantage in clinical applications, as shown in Table 19 and Figure 7.

**Example 24**

Inhibition of growth of xenograft tumors from human breast cancer MCF-7 cells in nude mice by intravenous injection of deuterium oxide in combination with gemcitabine

Animal: 6 to 7 week-aged female BALB/c nude mice, each weighing 18 to 20 g, provided by Shanghai SLAC Laboratory Animal Co. Ltd.

Cell lines: human breast cancer MCF-7 cells were provided by NANJING KEYGEN BIOTECH. CO., LTD.

Test drugs: gemcitabine (GEM) and the sodium chloride deuterium oxide solution prepared in Example 1.

Experimental method:

1. Modeling

Tumor cells were resuscitated and cultured according to a routine procedure, suspension of cultured MCF-7 cells was collected, sterile physiological saline was added thereto to make a suspension at a concentration of $1 \times 10^7$ cells/ml, and 0.1 ml of the suspension was subcutaneously inoculated into the right axillary fossa of each nude mouse.

2. Grouping and dosing scheme

The diameter of xenograft tumors in the nude mice was measured with a vernier caliper, and 10 days after inoculation, when tumors grew to 100 to 110 cm$^3$, the animals were randomized with 8 mice per group. Meanwhile drugs were administered by injection into tail vein, once every three days, for 21 successive days. By measuring the tumor diameter, the anti-tumor efficacy of test samples was dynamically observed. Meanwhile the body weight of animals was also recorded to observe the toxicity of the test samples. 21 days after administration, the nude mice were sacrificed, and tumors were harvested by surgery, weighed and photographed. Bone marrow nucleated cells were isolated by a routine method, DNA was extracted from the cells, the OD value was measured in a UV-260 spectrometer as a representative of the DNA content, and inhibition of DNA synthesis by the test samples was observed.

The test used 7 groups in total, with 8 animals per group.

The test used 7 groups:

1) Negative control group: physiological saline, 10 ml/kg;
2) Paclitaxel positive control group: paclitaxel diluted in physiological saline (8 mg/kg);
3) Gemcitabine positive control group: gemcitabine diluted in physiological saline (20 mg/kg);
4) Low-dose gemcitabine control group: gemcitabine diluted in physiological saline (10 mg/kg);
5) Test group 1: gemcitabine (10 mg/kg) diluted in the sodium chloride deuterium oxide solution (5 ml/kg).
6) Test group 2: gemcitabine (10 mg/kg) diluted in the sodium chloride deuterium oxide solution (10 ml/kg).
7) Test group 3: gemcitabine (10 ml/kg) diluted in the sodium chloride deuterium oxide solution (20 ml/kg).

3. Observation of indicators: the same as Example 23.

4. Statistics: the same as Example 23.

5. Results

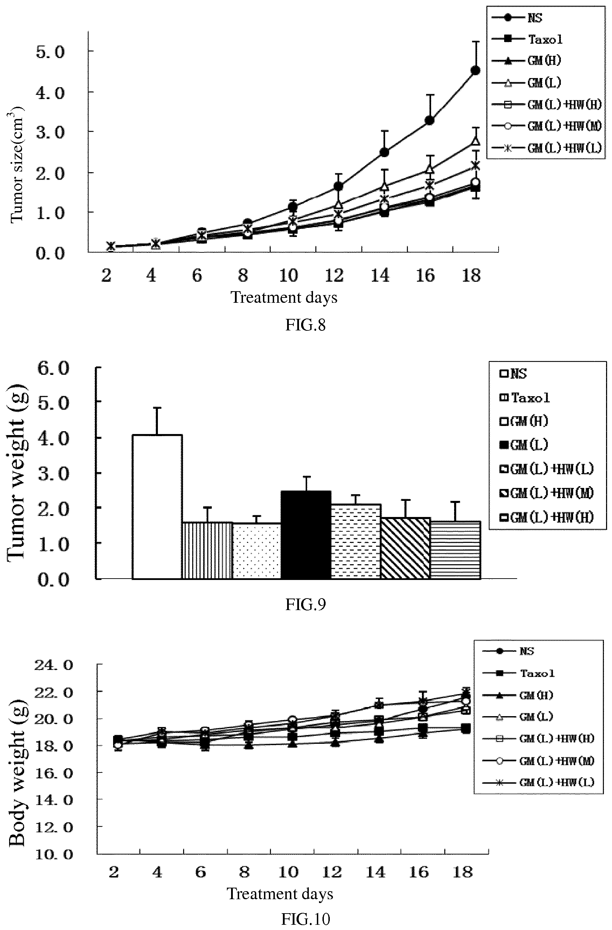

**Anti-tumor activity evaluation by xenograft tumor volume:** the sodium chloride deuterium oxide solution in combination with low-dose gemcitabine showed a good inhibitory effect on the xenograft tumors; 21 days after administration, the tumor volumes were 1.949±0.491 cm$^3$ in the group receiving the sodium chloride deuterium oxide solution (5 mg/kg) in combination with gemcitabine, 1.743±0.503 cm$^3$ in the group receiving the sodium chloride deuterium oxide solution (10 mg/kg) in combination with gemcitabine, and 1.671±0.386 cm$^3$ in the group receiving the sodium chloride deuterium oxide solution (20 mg/kg) in combination with gemcitabine, all with a T/C<40% as compared to the negative control group, and meeting the criteria for pharmacological efficacy. The group receiving low-dose gemcitabine alone showed only a slightly reduced tumor volume of 2.757±0.342 cm$^3$ with a T/C of 60.39%, which is greater than 40% and does not meet the criteria for pharmacological efficacy. When combined with gemcitabine, deuterium oxide significantly enhances the anti-tumor efficacy of gemcitabine, as shown in Figure 8.

**Anti-tumor activity evaluation by xenograft tumor weight:** the sodium chloride deuterium oxide solution in combination with low-dose gemcitabine showed a good inhibitory effect on the xenograft tumors; 21 days after administration, the inhibitions were 48.82% in the group receiving the sodium chloride deuterium oxide solution (5 mg/kg) in combination with gemcitabine, 57.78% in the group receiving the sodium chloride deuterium oxide solution (10 mg/kg) in combination with gemcitabine, and 60.70% in the group receiving the sodium chloride deuterium oxide solution (20 mg/kg) in combination with gemcitabine, all with a T/C>40% and P<0.001 as compared to the negative control group, and all meeting the criteria for pharmacological efficacy. The group receiving low-dose gemcitabine alone showed only a slightly reduced tumor weight and an inhibition of 39.18%, which is less than 40% and does not meet the criteria for pharmacological efficacy. Therefore, deuterium oxide significantly enhances the anti-tumor efficacy of gemcitabine, as shown in Table 20 and Figure 9.

Table 20. Effect of deuterium oxide in combination with gemcitabine on xenograft tumors from human breast cancer MCF-7 cells in nude mice ($\bar{x}\pm$SD n=8)

| Group | Dose × Times | Body weight (g) (Initial/End) | Tumor weight (g) | Inhibition (%) | Bone marrow DNA content (OD) |
|---|---|---|---|---|---|
| Negative Control NS | 10 ml/kg ×11 | 18.3/+3.3 | 4.07±0.76 | -- | 0.931±0.2011 |
| Paclitaxel positive control Taxol | 8mg/kg×11 | 18.3/+1.0 | 1.59±0.40 | 60.93%* | 0.881±0.2502 |
| Gemcitabine positive control GEM(H) | 20 mg/kg ×11 | 18.1/+0.9 | 1.57±0.21 | 61.42%* | 0.823±0.1416** |
| Gemcitabine low-dose GEM(L) | 10 mg/kg ×11 | 18.0/ +2.9 | 2.48±0.41 | 39.06% | 0.908±0.2543 |
| Test group 1 GEM (L)+HW(L) | NaCl deuterium oxide solution (5 ml/kg) + GEM(10 mg/kg)× 11 | 18.4/ +3.1 | 2.08±0.28 | 48.89%* | 0.923±0.3101 |
| Test group 2 GEM (L)+HW(M) | NaCl deuterium oxide solution (10 ml/kg) + GEM(10 mg/kg)× 11 | 18.0/ +3.3 | 1.72±0.51 | 57.73%* | 0.942±0.2896 |
| Test group 3 GEM (L)+HW(H) | NaCl deuterium oxide solution (20 ml/kg) + GEM(10 mg/kg)× 11 | 18.4/+2.4 | 1.6±0.55 | 60.68%* | 0.920±0.4167 |
| Compared to the negative control group: * P<0.001, ** P<0.05. Inhibition of tumor growth (%) > 40% indicates pharmacological efficacy. | | | | | |

**Toxicity reduction:** importantly, during the experiment, the average body weight of tumor-bearing mice in the negative control group increased by 3.3 g; in the paclitaxel and gemcitabine positive control groups, despite apparent reduction in tumor weight, the body weight of tumor-bearing mice only slightly increased, and the bone marrow DNA content also only slightly increased, indicating certain toxicity of paclitaxel and gemcitabine to animals. The sodium chloride deuterium oxide solution in combination with gemcitabine (10 mg/kg, half of that of control) can achieve the same efficacy as high-dose paclitaxel (8 mg/kg) and high-dose gemcitabine (20 mg/kg) (equivalence), while the body weight of the tumor-bearing mice increased similarly to the negative control group, and the bone marrow DNA content also increased, without the toxicity caused by high-dose paclitaxel and gemcitabine. Toxicity of anti-tumor drugs is very harmful to patients and even unacceptable to some weak or aged patients, and thus causes a serious problem to clinical anti-tumor chemotherapy. Hence, deuterium oxide in combination with low-dose gemcitabine has a great advantage in clinical applications, as shown in Table 20 and Figure 10.

5-FU and gemcitabine are antimetabolites among cytotoxic anti-tumor drugs. It can be seen in Examples 23 and 24 that deuterium oxide in combination with antimetabolite anti-tumor drugs has general applicability for inhibition of growth of various tumors.

**Example 25**

Inhibition of growth of xenograft tumors from human lung cancer A549 cells in nude mice by intravenous injection of deuterium oxide in combination with gemcitabine

Animal: 6 to 7 week-aged female BALB/c nude mice, each weighing 18 to 20 g, provided by Shanghai SLAC Laboratory Animal Co. Ltd.

Cell lines: human lung cancer A549 cells were provided by NANJING KEYGEN BIOTECH. CO., LTD.

Test drugs: gemcitabine (GEM) and the sodium chloride deuterium oxide solution prepared in Example 1.

1. Tumor cells were resuscitated and inoculated, and sterile physiological saline was added thereto to make a cell suspension which was subcutaneously inoculated into the upper right axillary fossa of animals. $5 \times 10^6$ tumor cells were inoculated into each animal.

2. Grouping and dosing scheme

The diameter of xenograft tumors in the nude mice was measured with a vernier caliper, and 10 days after inoculation, when tumors grew to 100 to 110 cm$^3$, the animals were randomized with 8 mice per group. Meanwhile drugs were administered by injection into tail vein, once every three days, for 21 successive days. By measuring the tumor diameter, the anti-tumor efficacy of test samples was dynamically observed. Meanwhile the body weight of animals was also recorded to observe the toxicity of the test samples. 21 days after administration, the nude mice were sacrificed, and tumors were harvested by surgery, weighed and photographed.

The test used 7 groups:

1) Physiological saline control group: physiological saline, 10 ml/kg;
2) Paclitaxel positive control group: paclitaxel diluted in physiological saline (8 mg/kg);
3) Gemcitabine positive control group: gemcitabine diluted in physiological saline (20 mg/kg);
4) Low-dose gemcitabine control group: gemcitabine diluted in physiological saline (10 mg/kg);
5) Test group 1: gemcitabine (10 mg/kg) diluted in the sodium chloride deuterium oxide solution (5 ml/kg).
6) Test group 2: gemcitabine (10 mg/kg) diluted in the sodium chloride deuterium oxide solution (10 ml/kg).
7) Test group 3: gemcitabine (10 ml/kg) diluted in the sodium chloride deuterium oxide solution (20 ml/kg).

3. Observation of indicators: the same as Example 23.

4. Statistics: the same as Example 23.

5. Results

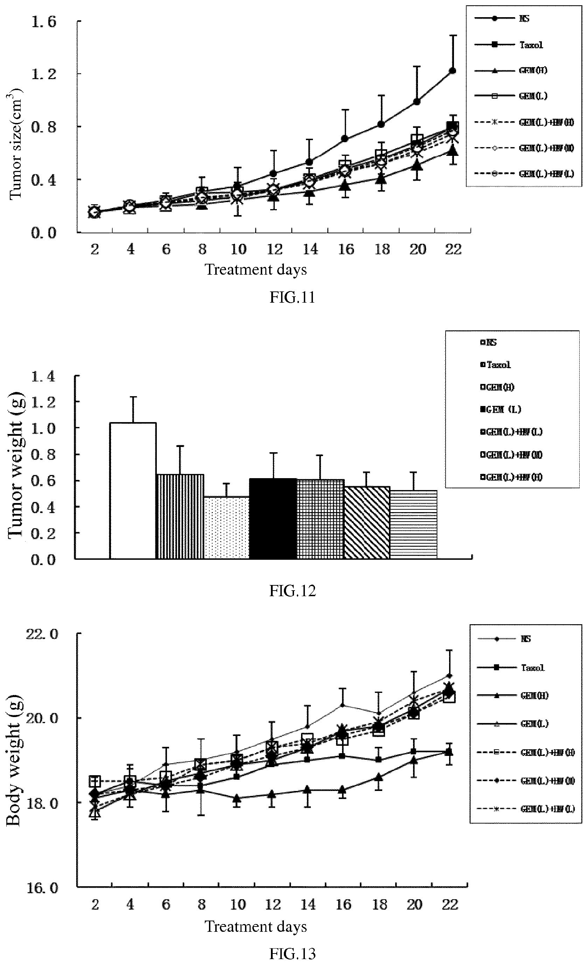

**Anti-tumor activity evaluations by xenograft tumor volume and weight:** the results demonstrate that, 21 days after administration, as compared to the physiological saline control group, the group receiving low-dose gemcitabine alone showed a reduced tumor weight to 2.23 ± 0.2 g and an inhibition of 23.6%, which however does not meet the criteria for pharmacological efficacy. As compared to the physiological saline control group, the sodium chloride deuterium oxide solutions in combination with gemcitabine all showed a good inhibitory effect on the xenograft tumors; the group receiving the sodium chloride deuterium oxide solution (5 ml) in combination with gemcitabine showed a tumor weight of 1.34±0.38 g and a tumor proliferation ratio of 54.22%, the group receiving the sodium chloride deuterium oxide solution (10 ml) in combination with gemcitabine showed a tumor weight of 1.35±0.34 g and a tumor proliferation ratio of 53.70%, and the group receiving the sodium chloride deuterium oxide solution (20 ml) in combination with gemcitabine showed a tumor weight of 1.28±0.46 g and a tumor proliferation ratio of 56.32%, all having P<0.001 and meeting the criteria for pharmacological efficacy, as shown in Figures 11 and 12.

Table 21. Effect of sodium chloride injection solutions in deuterium oxide on tumor weight of human lung cancer xenograft tumors in nude mice ($\bar{x}$±SD, n=8)

| Group | Dose × Times | Body weight (g) (Initial/End) | Tumor weight (g) | Inhibition (%) |
|---|---|---|---|---|
| Physiological saline control | 10 ml/kg | 18.5/+1.4 | 2.92±0.27 | -- |

(continued)

| Group | Dose × Times | Body weight (g) (Initial/End) | Tumor weight (g) | Inhibition (%) |
|---|---|---|---|---|
| Paclitaxel positive control Taxol | 8mg/kg×11 | 18.4/+0.9 | 0.94 ± 0.25 | 67.97* |
| Gemcitabine positive control GEM(H) | 20 mg/kg × 11 | 18.4/+0.5 | 1.04±0.13 | 64.54* |
| Gemcitabine low-dose GEM(L) | 10 mg/kg × 11 | 18.0/ +0.4 | 2.23 ± 0.2 | 23.64 |
| Test group 1 GEM(L) +HW(L) | NaCl deuterium oxide solution (5 ml/kg) + GEM(10 mg/kg) × 11 | 18.3/+1.5 | 1.34±0.38 | 54.22* |
| Test group 2 GEM(L) +HW(M) | NaCl deuterium oxide solution (10 ml/kg) + GEM(10 mg/kg)× 11 | 18.5/+1.1 | 1.35±0.34 | 53.70* |
| Test group 3 GEM (L) +HW(H) | NaCl deuterium oxide solution (20 ml/kg) + GEM(10 mg/kg)× 11 | 18.4/+0.7 | 1.28±0.46 | 56.32* |
| As compared to the control group, *P<0.001, and inhibition of tumor weight > 40% indicates pharmacological efficacy. | | | | |

Importantly, in the paclitaxel and gemcitabine positive control groups, despite an apparent reduction in tumor weight to $0.94 \pm 0.25$ g and $1.04 \pm 0.13$ g, respectively, and a tumor proliferation ratio of 67.97% and 64.54%, respectively, the body weight of tumor-bearing mice barely increased, indicating significant toxicity of paclitaxel and gemcitabine to animals. Deuterium oxide in combination with low-dose gemcitabine can achieve the same efficacy as high-dose paclitaxel and gemcitabine, does not cause the corresponding toxicity, and results in an increased body weight of tumor-bearing mice during administration, as shown in Table 21 and Figure 13.

**Example 26**

Inhibition of growth of xenograft tumors from human ovarian cancer SKOV3 cells in nude mice by intravenous injection of deuterium oxide in combination with paclitaxel
Animal: 6 to 7 week-aged female BALB/c nude mice, each weighing 18 to 20 g, provided by Shanghai SLAC Laboratory Animal Co. Ltd.
Cell lines: human ovarian cancer SKOV3 cells were provided by NANJING KEYGEN BIOTECH. CO., LTD.
Test drugs: paclitaxel and the sodium chloride deuterium oxide solution prepared in Example 1.

Experimental method:

1. Tumor cell resuscitation and inoculation

Tumor cells were resuscitated and inoculated, and sterile physiological saline was added thereto to make a cell suspension which was subcutaneously inoculated into the upper right axillary fossa of animals. $5 \times 10^6$ tumor cells were inoculated into each animal.

2. Grouping and dosing scheme

The diameter of xenograft tumors in the nude mice was measured with a vernier caliper, and 10 days after inoculation, when tumors grew to 100 to 110 cm$^3$, the animals were randomized. Meanwhile drugs were administered by injection into tail vein, once every three days, for 21 successive days. The body weight of animals was also recorded to observe the toxicity of the test samples. 21 days after administration, the nude mice were sacrificed, and tumors were harvested by surgery, and weighed.
The test used 6 groups in total, with 5 animals per group.

1) Physiological saline control group: physiological saline, 10 ml/kg;
2) Paclitaxel positive control group: paclitaxel diluted in physiological saline (8 mg/kg);
3) Sodium chloride deuterium oxide solution (10 ml/kg);
4) Test group 1: paclitaxel (8 mg/kg) diluted in the sodium chloride deuterium oxide solution (5 ml/kg);
5) Test group 2: paclitaxel (8 mg/kg) diluted in the sodium chloride deuterium oxide solution (10 ml/kg);
6) Test group 3: paclitaxel (8 mg/kg) diluted in the sodium chloride deuterium oxide solution (20 ml/kg).

3. Observation of indicators: the same as Example 23.

4. Statistics: the same as Example 23.

The results are shown in Table 22.

Table 22. Effect of sodium chloride deuterium oxide solution in combination with paclitaxel on tumor weight of xenograft tumors from human ovarian cancer SKOV3 cells in nude mice ($\bar{x} \pm$ SD, n=5)

| Group | Dose × Times | Body weight (g) (Initial/End) | Tumor weight (g) | Inhibition (%) |
|---|---|---|---|---|
| Physiological saline control | 10 ml/kg | 20.8/+4.0 | 3.22±1.59 | -- |
| Paclitaxel | 8mg/kg×11 | 20.1/+2.8 | 2.07±1.06 | 35.7 |
| NaCl deuterium oxide solution | 10ml/kg× 11 | 21.1/+3.9 | 2.44±1.51 | 24.2 |
| Test group 1 NaCl deuterium oxide solution + Paclitaxel | NaCl deuterium oxide solution (5ml/kg) + Paclitaxel (8mg/kg) ×11 | 21.0/+3.8 | 1.95±0.57 | 39.4 |
| Test group 2 NaCl deuterium oxide solution + Paclitaxel | NaCl deuterium oxide solution (10ml/kg) + Paclitaxel (8mg/kg) × 11 | 20.4/+3.7 | 1.71±0.87 | 46.8* |
| Test group 3 NaCl deuterium oxide solution + Paclitaxel | NaCl deuterium oxide solution (20ml/kg) + Paclitaxel (8mg/kg) × 11 | 20.9/+4.1 | 1.45±0.42 | 54.9* |
| Compared to the control group: * P<0.01. | | | | |

Paclitaxel is an important phytogenic anti-tumor drug, having various types of a similar mechanism of action, including paclitaxel, paclitaxel liposomes, paclitaxel albumin, and docetaxel. The results demonstrate that, 21 days after administration, as compared to the model group (the physiological saline group), the groups receiving the sodium chloride deuterium oxide solutions (10 ml/kg and 20 ml/kg) in combination with paclitaxel (8 mg/kg) showed a good inhibitory effect on xenograft tumors from human ovarian cancer SKOV3 cells in tumor-bearing nude, with a significant reduction in tumor weight, while the tumor inhibition T/C was 54.9% on day 21 with P<0.001. On the other hand, as compared to the model group, the group receiving paclitaxel (8 mg/kg) alone showed a reduction, but did not meet the criteria of >40% for pharmacological efficacy. As compared to the model group (the physiological saline group), the group receiving the sodium chloride deuterium oxide solutions (5 ml/kg) in combination with paclitaxel (12 mg/kg) showed a reduction, but did not meet the criteria of >40% for pharmacological efficacy. Deuterium oxide as a broad-spectrum anti-tumor efficacy-enhancing agent can also enhance the efficacy of phytogenic anti-tumor drugs.

**Example 27**

Inhibition of growth of xenograft tumors from human bladder cancer 5637 cells in nude mice by intravenous injection of deuterium oxide in combination with thiotepa
Animal: 6 to 7 week-aged female BALB/c nude mice, each weighing 18 to 20 g, provided by Shanghai SLAC Laboratory Animal Co. Ltd.
Cell lines: human bladder cancer 5637 cells were provided by NANJING KEYGEN BIOTECH. CO., LTD.
Test drugs: thiotepa and the sodium chloride deuterium oxide solution prepared in Example 1.

Experimental method:

1. Tumor cell resuscitation and inoculation

Tumor cells were resuscitated and inoculated, and sterile physiological saline was added thereto to make a cell suspension which was subcutaneously inoculated into the upper right axillary fossa of animals. $5 \times 10^6$ tumor cells were inoculated into each animal.

2. Grouping and dosing scheme

The diameter of xenograft tumors in the nude mice was measured with a vernier caliper, and 10 days after inoculation, when tumors grew to 100 to 110 cm$^3$, the animals were randomized. Meanwhile drugs were administered by injection into tail vein, once every three days, for 21 successive days. The body weight of animals was also recorded to observe the toxicity of the test samples. 21 days after administration, the nude mice were sacrificed, and tumors were harvested by surgery, and weighed.
The test used 6 groups in total, with 5 animals per group.

   1) Physiological saline control group: physiological saline, 10 ml/kg;
   2) Thiotepa positive control group: thiotepa diluted in physiological saline (0.2 mg/kg);
   3) Test group 1: thiotepa (0.2 mg/kg) diluted in the sodium chloride deuterium oxide solution (1 ml/kg);
   4) Test group 2: thiotepa (0.2 mg/kg) diluted in the sodium chloride deuterium oxide solution (10 ml/kg);
   5) Test group 3: thiotepa (0.2 mg/kg) diluted in the sodium chloride deuterium oxide solution (20 ml/kg).

3. Observation of indicators: the same as Example 23.

4. Statistics: the same as Example 23.

The results demonstrate that, 21 days after administration, as compared to the model group (the physiological saline group), the groups receiving the two dosages of sodium chloride deuterium oxide solutions (10 ml/kg and 20 ml/kg) in combination with thiotepa (0.2 mg/kg) showed a good inhibitory effect on xenograft tumors from human bladder cancer 56373 cells in tumor-bearing nude, with a significant reduction in tumor weight, while the tumor inhibitions T/C were 47.0% and 63.4% on day 21, with P<0.01 and P<0.001, respectively. On the other hand, as compared to the model group, the group receiving thiotepa (0.2 mg/kg) alone did not show a considerable difference. As compared to the model group (the physiological saline group), the group receiving the sodium chloride deuterium oxide solutions (5 ml/kg) in combination with thiotepa (0.2 mg/kg) did not show a considerable difference. The results are shown in Table 23.

Table 23. Effect of sodium chloride deuterium oxide solution in combination with thiotepa on tumor weight of xenograft tumors from human bladder cancer 56373 cells in nude mice ($\bar{x} \pm$SD, n=5)

| Groups | Dose × Times | Body weight (g) (Initial/End) | Tumor weight (g) | Inhibition (%) |
|---|---|---|---|---|
| Physiological saline control | 10 ml/kg | 19.8/+3.1 | 2.87±1.11 | -- |
| Thiotepa | 0.2mg/kg× 11 | 20.6/+2.6 | 1.75±1.06 | 39.0 |
| Test group 1 NaCl deuterium oxide solution + thiotepa | NaCl deuterium oxide solution (1ml/kg) + thiotepa (0.2mg/kg) ×11 | 20.4/+3.3 | 1.72±0.73 | 40.1 |
| Test group 2 NaCl deuterium oxide solution + thiotepa | NaCl deuterium oxide solution (10ml/kg) + thiotepa (0.2mg/kg) × 11 | 20.1/+3.0 | 1.32±0.55 | 54.0* |
| Test group 3 NaCl deuterium oxide solution + thiotepa | NaCl deuterium oxide solution (20ml/kg) + thiotepa (0.2mg/kg) × 11 | 19.9/+4.1 | 1.05±0.38 | 63.4** |
| Compared to the control group: * P<0.01, ** P<0.001. | | | | |

Thiotepa is an ethyleneimine alkylating agent involved in guanine binding and affecting DNA synthesis, and is effective on various types of tumor cells, especially bladder cancer cells. Deuterium oxide in combination with thiotepa can synergistically enhance the effect of thiotepa against bladder cancer cells 56373, increasing the inhibition of cancer cell growth from 39% (when thiotepa is used alone) to 54%-63.4%, which verifies the function of deuterium oxide as a broad-spectrum anti-tumor efficacy-enhancing agent. However, when the dose of deuterium oxide is very low (1 mg/kg), the effect is not significant.

**Example 28**

Inhibition of microvessel density of xenograft tumors from human pancreatic cancer PANC-1 cells in nude mice by intravenous injection of deuterium oxide in combination with bevacizumab
Animal: 6 to 7 week-aged female BALB/c nude mice, each weighing 18 to 20 g, provided by Shanghai SLAC Laboratory Animal Co. Ltd.
Cell lines: human pancreatic cancer PANC-1 cells were provided by NANJING KEYGEN BIOTECH. CO., LTD.
Test drugs: bevacizumab (Avastin, Roche) and the sodium chloride deuterium oxide solution prepared in Example 1.

Experimental method:

1. Modeling

Tumor cells were resuscitated and cultured according to a routine procedure, suspension of cultured PANC-1 cells was collected, sterile physiological saline was added thereto to make a suspension at a concentration of $1\times10^7$ cells/ml, and 0.1 ml of the suspension was subcutaneously inoculated into the right axillary fossa of each nude mouse.

2. Grouping and dosing scheme

The diameter of xenograft tumors in the nude mice was measured with a vernier caliper, and 10 days after inoculation, when tumors grew to 100 to 110 cm$^3$, the animals were randomized. Meanwhile drugs were administered by injection into tail vein, once every three days, for 14 successive days. The body weight of animals was also recorded to observe the toxicity of the test samples. 14 days after administration, the dosing was discontinued for 1 week, then the nude mice were sacrificed, tumors were harvested by surgery and weighed, and the microvessel density (MVD) was examined. Tumors were stained by a standard EnVisionTM method, the number of microvessels was counted by the Weidner microvessel method, the entire field was observed under 100× magnification, the areas having the highest microvessel density of the tumor were selected for counting, the microvessel density in three magnified fields was counted under 400× magnification, and the obtained values of microvessel density were averaged as the MVD.
The test used 4 groups in total, with 5 animals per group.

    1) Negative control group: 0.9% sodium chloride injection, 10 ml/kg;
    2) Bevacizumab positive control group: bevacizumab (5 mg/kg) diluted in 0.9% sodium chloride injection;
    3) Test group 1: bevacizumab (5 mg/kg) diluted in the sodium chloride deuterium oxide solution (10 ml/kg);
    4) Test group 2: bevacizumab (5 mg/kg) diluted in the sodium chloride deuterium oxide solution (20 ml/kg).

3. Observation of indicators: the same as Example 23.

4. Statistics: the same as Example 23.

5. Results: The results are shown in Tables 24 and 25.

Table 24. Effect of deuterium oxide in combination with bevacizumab on tumor weight of xenograft tumors ($\bar{x}\pm$SD, n=5)

| Groups | Tumor weight (g) | Inhibition of growth (%) |
| --- | --- | --- |
| Negative control | 1.97±0.29 | |
| Bevacizumab positive control | 11.12±0.17* | 43.1 |

(continued)

| Groups | Tumor weight (g) | Inhibition of growth (%) |
|---|---|---|
| Bevacizumab (5 mg/kg) diluted in sodium chloride deuterium oxide solution (10 ml/kg) | 1.04±0.13* | 47.2 |
| Bevacizumab (5 mg/kg) diluted in sodium chloride deuterium oxide solution (20 ml/kg) | 0.93±0.2* | 53.7 |
| Compared to the negative control group: * P<0.01. | | |

Table 25. Effect of deuterium oxide in combination with bevacizumab on microvessel density (MVD) ($\bar{x}\pm$SD, n=5)

| Groups | MVD |
|---|---|
| Negative control | 31.10±4.47 |
| Bevacizumab positive control | 22.40±3.22* |
| Bevacizumab (5 mg/kg) diluted in sodium chloride deuterium oxide solution (10 ml/kg) | 18.80±1.58* |
| Bevacizumab (5 mg/kg) diluted in sodium chloride deuterium oxide solution (20 ml/kg) | 16.60±1.92* |
| Compared to the negative control group: * P<0.05. | |

Growth of tumor tissue requires a large amount of blood supply, and bevacizumab is an angiogenesis inhibitor. When the growth of microvessels in tumor tissue is inhibited, microvessels and blood supply are reduced. The results demonstrate that deuterium oxide as a broad-spectrum anti-tumor efficacy-enhancing agent can also enhance the effect of bevacizumab in reducing the microvessel density (MVD) in tumor tissue, reducing the blood supply to pancreatic cancer PANC-1 cells, and in turn inhibiting tumor growth.

**Example 29**

Studies on drug safety: toxicity test for intravenous injection of sodium chloride deuterium oxide solution

Experimental method:

6 to 7 week-aged SD rats, half male and half female, with males each weighing 280 to 338 g and females each weighing 212 to 268 g, were provided by Beijing Vital River Laboratory Animal Technology Co., Ltd.
Test sample: the sodium chloride deuterium oxide solution prepared in Example 1.

Experiment 1: Observation of toxicity after single dosing

Dosing scheme: A maximum dosing method was used, in which the sodium chloride deuterium oxide solution prepared in Example 1 was intravenously injected at a dose of 2 ml/100 g into 20 rats, half male and half female, at an injection speed of 2 ml/min. An equal volume of a 0.9% sodium chloride rejection was given to the control group containing 10 rats, half male and half female.

Table 26. Dosing and grouping.

| Groups | Dose (mg/kg) | Drug volume (ml/100g) | Number of animals | Serial No. of Male | Serial No. of Female |
|---|---|---|---|---|---|
| NaCl deuterium oxide solution | --- | 2 | 20 | 1101-1110 | 2101-2110 |
| Control | 0* | 2 | 10 | 1001-1005 | 2001-2005 |
| * indicates that an equal volume of a 0.9% sodium chloride rejection was given to the control group. | | | | | |

Experimental results:

1. Observation of general symptoms

In the toxicity experiment in which a single dose of sodium chloride deuterium oxide solution was intravenously administered to rats, a maximum dose of 20 ml/kg was given to the subjects. Immediately after administration, animals receiving the test sample all showed symptoms such as reduced activity, shortness of breath, and increased urination, which were recovered to normal within 1 hour after the administration, and no other apparent abnormalities were found.
Observations were made at least once a day for 14 successive days, and no abnormalities in appearance and body sign, behavior and activity, and excrement characteristics or death was seen.

2. Body weight

During the experiment, the animals showed a good general status and a normal increase in body weight.

Table 27. Body weight change of rats (male) in the toxicity experiment with single intravenous administration of sodium chloride deuterium oxide solution (g, $\bar{x}\pm S$)

| Groups | 0 day(s) post administration | 7 day(s) post administration | 14 day(s) post administration |
|---|---|---|---|
| NaCl deuterium oxide solution | 304.40±21.10 | 333.00±21.47 | 360.50±16.30 |
| Control | 309.40±10.09 | 334.80±9.58 | 364.40±11.19 |
| Note: the group receiving sodium chloride deuterium oxide solution: n=10; the control group: n=5. | | | |

Table 28. Body weight change of rats (female) in the toxicity experiment with single intravenous administration of sodium chloride deuterium oxide solution (g, $\bar{x}\pm S$)

| Groups | 0 day(s) post administration | 7 day(s) post administration | 14 day(s) post administration |
|---|---|---|---|
| NaCl deuterium oxide solution | 248.80±17.08 | 264.20±19.58 | 275.80±19.45 |
| Control | 250.20±6.22 | 267.60±10.97 | 278.40±11.72 |
| Note: the group receiving sodium chloride deuterium oxide solution: n=10; the control group: n=5. | | | |

Discussion: in the toxicity experiment in which a single dose of sodium chloride deuterium oxide solution as the test sample was intravenously administered to rats, a maximum dose of 20 ml/kg of the sodium chloride deuterium oxide solution was given to the subjects. Immediately after administration, symptoms such as reduced activity and shortness of breath were observed, and no other toxicities associated with the test sample were found. Observations were continued for two weeks, the animals showed a normal increase in body weight, and no death was seen. According to the results of this experiment, for single-dose intravenous administration to rats, the minimum lethal dose (MLD) of the sodium chloride deuterium oxide solution as the test sample is greater than 20 ml/kg.

Experiment 2: Observation of toxicity during multiple dosing

Dosing scheme: A method of repeatedly administrating the maximum dose was used, in which the sodium chloride deuterium oxide solution prepared in Example 1 was intravenously injected once a day at a dose of 2 ml/100 g and an injection speed of 2 ml/min to 20 rats, half male and half female, for 5 successive days. An equal volume of a 0.9% sodium chloride rejection was given to the control group containing 10 rats, half male and half female.

Table 29. Dosing and grouping.

| Groups | Dose (mg/kg) | Drug volume (ml/100g) | Number of animals | Serial No. of Male | Serial No. of Female |
|---|---|---|---|---|---|
| NaCl deuterium oxide solution | --- | 2 x 5 times | 20 | 1201-1210 | 2201-2210 |

(continued)

| Groups | Dose (mg/kg) | Drug volume (ml/100g) | Number of animals | Serial No. of Male | Serial No. of Female |
|---|---|---|---|---|---|
| Control | 10* | 2 x 5 times | 10 | 1101-1105 | 2101-2105 |
| * indicates that an equal volume of a 0.9% sodium chloride rejection was given to the control group. | | | | | |

Experimental results:

1. Observation of general symptoms

In the toxicity experiment in which multiple doses of sodium chloride deuterium oxide solution were intravenously administered to rats, a maximum dose of 20 ml/kg was given to the subjects once a day, for 5 successive days. Immediately after each administration, animals receiving the test sample all showed symptoms such as reduced activity, shortness of breath, and increased urination, which were recovered to normal within 1 hour after the administration, and no other apparent abnormalities were found.

Observations were made at least once a day for 3 successive months, and no abnormalities in appearance and body sign, behavior and activity, and excrement characteristics or death was seen.

2. Body weight

During the experiment, the animals showed a good general status and a normal increase in body weight.

Table 30. Body weight change of rats (male) in the toxicity experiment with multiple repeating intravenous administrations of sodium chloride deuterium oxide solution (g, $\bar{x}\pm S$)

| Groups | 0 day(s) post administration | 30 day(s) post administration | 90 day(s) post administration |
|---|---|---|---|
| NaCl deuterium oxide solution | 305.20±20.90 | 352.00±31.72 | 452.40±30.14 |
| Control | 303.20±11.10 | 361.01±29.65 | 461.91±31.19 |
| Note: the group receiving sodium chloride deuterium oxide solution: n=10; the control group: n=5. | | | |

Table 31. Body weight change of rats (female) in the toxicity experiment with multiple repeating intravenous administrations of sodium chloride deuterium oxide solution (g, $\bar{x}\pm S$)

| Groups | 0 day(s) post administration | 30 day(s) post administration | 90 day(s) post administration |
|---|---|---|---|
| NaCl deuterium oxide solution | 243.52±14.88 | 293.20±18.83 | 395.63±29.54 |
| Control | 251.28±11.22 | 298.12±14.67 | 405.42±26.12 |
| Note: the group receiving sodium chloride deuterium oxide solution: n=10; the control group: n=5. | | | |

In the toxicity experiment in which multiple doses of sodium chloride deuterium oxide solution as the test sample were repeatedly intravenously administered to rats, a maximum dose of 20 ml/kg of the sodium chloride deuterium oxide solution was given to the subjects. Immediately after administration, symptoms such as reduced activity and shortness of breath were observed, and no other toxicities associated with the test sample were found. Observations were continued for 3 months, the animals showed a normal increase in body weight, and no death was seen. According to the results of this experiment, for multiple-dose intravenous administration to rats, the minimum lethal dose (MLD) of the sodium chloride deuterium oxide solution as the test sample is greater than 20 ml/kg.

Although some aspects of the present invention have been set forth and discussed herein, it would be appreciated by a person skilled in the art that modifications can be made to the above aspects within the scope of the appended claims.

**Claims**

1. A pharmaceutical solution for use in enhancing anti-tumor efficacy and in reducing toxicity of an anti-tumor drug, when used in combination with at least one anti-tumor drug in the treatment or prevention of malignant tumors, wherein the pharmaceutical solution contains deuterium oxide as a solvent, wherein every 100 ml of the pharmaceutical solution contain 95 to 99.9 ml deuterium oxide, and in the deuterium oxide the isotope abundance of deuterium is 90% to 99.9%, and wherein the anti-tumor drug is selected from the group consisting of 5-fluorouracil, gemcitabine, mitomycin, oxaliplatin, paclitaxel, thiotepa and bevacizumab.

2. The pharmaceutical solution for use according to Claim 1, **characterized in that** the pharmaceutical solution comprises sodium chloride, being a sodium chloride deuterium oxide solution containing 0.1 g to 5 g sodium chloride, preferably 0.5 g to 2.5 g sodium chloride per 100 ml pharmaceutical solution, most preferably being a sodium chloride deuterium oxide solution containing 0.9 g sodium chloride per 100 ml pharmaceutical solution which is prepared with deuterium oxide and sodium chloride and has a pH adjusted to 4.5 to 7.0.

3. The pharmaceutical solution for use according to Claim 1, **characterized in that**, the pharmaceutical solution comprises glucose, being a glucose deuterium oxide solution containing 0.1 g to 50 g glucose, preferably 5 to 50 g per 100 ml pharmaceutical solution, most preferably being a glucose deuterium oxide solution containing 5 g or 10 g glucose per 100 ml pharmaceutical solution which is prepared with deuterium oxide and glucose and has a pH adjusted to 3.2 to 6.5.

4. The pharmaceutical solution for use according to Claim 1, **characterized in that**, the pharmaceutical solution is a glucose-sodium chloride deuterium oxide solution containing 0.9 g sodium chloride and 5 g glucose per 100 ml pharmaceutical solution, which is prepared with deuterium oxide, sodium chloride and glucose and has a pH adjusted to 3.5 to 5.5.

5. The pharmaceutical solution for use according to Claim 1, **characterized in that**, the pharmaceutical solution is a Ringer's deuterium oxide solution containing 0.9 g sodium chloride, 0.012 g potassium chloride and 0.024 g calcium chloride per 100 ml pharmaceutical solution, which is prepared with deuterium oxide, sodium chloride, potassium chloride and calcium chloride and has a pH adjusted to 4.5 to 7.5.

6. The pharmaceutical solution for use according to Claim 1, **characterized in that**, the pharmaceutical solution is a solution of sodium bicarbonate in deuterium oxide containing 1 g to 50 g, preferably 5 g to 10 g, most preferably 5 g sodium bicarbonate per 100 ml pharmaceutical solution and having a pH adjusted to 7.5 to 8.5.

7. The pharmaceutical solution for use according to Claim 1, wherein the pharmaceutical solution is a solution for instillation and lavage, a solution for injection, a suspension, an emulsion, or a solution for embolization; and the solution for injection is preferably an intravenous injection, an intra-arterial injection, an intrathecal injection, or an intratumoral and peritumoral injection.

8. The pharmaceutical solution for use according to Claim 1, wherein in the deuterium oxide the isotope abundance of deuterium is 99.8% to 99.9%.

9. The pharmaceutical solution for use according to Claim 1, wherein the dose of the solution for injection is 1 ml/kg to 20 ml/kg.

10. The pharmaceutical solution for use according to Claim 1, wherein the tumor is selected from lung cancer, colorectal cancer, primary liver cancer, esophageal cancer, gastric cancer and cardiac cancer, pancreatic cancer, renal cell carcinoma, bladder cancer, prostate cancer, head and neck cancer, nasopharyngeal cancer, cervical cancer, ovarian cancer, breast cancer, brain tumor, bone and joint sarcoma, thyroid cancer, skin cancer, malignant melanoma, malignant lymphoma, leukemia, and complications and recurrence of various malignant tumors, such as recurrence, metastasis and implantation of tumor cells in thoracic, peritoneal, bladder and/or pelvic cavities, and malignant effusion in thoracic, peritoneal, bladder and/or pelvic cavities.

**Patentansprüche**

1. Pharmazeutische Lösung zur Verwendung in der Verbesserung der Antitumor-Wirksamkeit und in der Verringerung der Toxizität eines Antitumor-Arzneistoffs, wenn sie in Kombination mit zumindest einem Antitumor-Arzneistoff in der Behandlung oder Vorbeugung von bösartigen Tumoren verwendet wird, wobei die pharmazeutische Lösung Deuteriumoxid als ein Lösungsmittel enthält, wobei jeweils 100 ml der pharmazeutischen Lösung 95 bis 99,9 ml Deuteriumoxid enthalten, und in dem Deuteriumoxid die Isotopenhäufigkeit von Deuterium 90 % bis 99,9 % beträgt, und wobei der Antitumor-Arzneistoff ausgewählt ist aus der Gruppe bestehend aus 5-Fluorouracil, Gemcitabin, Mitomycin, Oxaliplatin, Paclitaxel, Thiotepa und Bevacizumab.

2. Pharmazeutische Lösung zur Verwendung nach Anspruch 1, **dadurch gekennzeichnet, dass** die pharmazeutische Lösung Natriumchlorid umfasst, und somit eine Natriumchlorid-Deuteriumoxid-Lösung ist, die 0,1 g bis 5 g Natriumchlorid, vorzugsweise 0,5 g bis 2,5 g Natriumchlorid je 100 ml pharmazeutischer Lösung enthält, am meisten bevorzugt eine Natriumchlorid-Deuteriumoxid-Lösung, die 0,9 g Natriumchlorid je 100 ml pharmazeutischer Lösung enthält, und die mit Deuteriumoxid und Natriumchlorid hergestellt wird und einen pH aufweist, der auf 4,5 bis 7,0 eingestellt ist.

3. Pharmazeutische Lösung zur Verwendung nach Anspruch 1, **dadurch gekennzeichnet, dass** die pharmazeutische Lösung Glucose umfasst, und somit eine Glucose-Deuteriumoxid-Lösung ist, die 0,1 g bis 50 g Glucose, vorzugsweise 5 bis 50 g Glucose je 100 ml pharmazeutischer Lösung enthält, am meisten bevorzugt eine Glucose-Deuteriumoxid-Lösung, die 5 g oder 10 g Glucose je 100 ml pharmazeutischer Lösung enthält, und die mit Deuteriumoxid und Glucose hergestellt wird und einen pH aufweist, der auf 3,2 bis 6,5 eingestellt ist.

4. Pharmazeutische Lösung zur Verwendung nach Anspruch 1, **dadurch gekennzeichnet, dass** die pharmazeutische Lösung eine Glucose-Natriumchlorid-Deuteriumoxid-Lösung ist, die 0,9 g Natriumchlorid und 5 g Glucose je 100 ml pharmazeutischer Lösung enthält, und die mit Deuteriumoxid, Natriumchlorid und Glucose hergestellt wird und einen pH aufweist, der auf 3,5 bis 5,5 eingestellt ist.

5. Pharmazeutische Lösung zur Verwendung nach Anspruch 1, **dadurch gekennzeichnet, dass** die pharmazeutische Lösung eine Ringer-Deuteriumoxid-Lösung ist, die 0,9 g Natriumchlorid, 0,012 g Kaliumchlorid und 0,024 g Kalziumchlorid je 100 ml pharmazeutischer Lösung enthält, und die mit Deuteriumoxid, Natriumchlorid, Kaliumchlorid und Kalziumchlorid hergestellt wird und einen pH aufweist, der auf 4,5 bis 7,5 eingestellt ist.

6. Pharmazeutische Lösung zur Verwendung nach Anspruch 1, **dadurch gekennzeichnet, dass** die pharmazeutische Lösung eine Lösung von Natriumbicarbonat in Deuteriumoxid ist, die 1 g bis 50 g, vorzugsweise 5 g bis 10 g, am meisten bevorzugt 5 g Natriumbicarbonat je 100 ml pharmazeutischer Lösung enthält und einen pH aufweist, der auf 7,5 bis 8,5 eingestellt ist.

7. Pharmazeutische Lösung zur Verwendung nach Anspruch 1, wobei die pharmazeutische Lösung eine Lösung zur Instillation und Lavage, eine Lösung zur Injektion, eine Suspension, eine Emulsion oder eine Lösung zur Embolisation ist; und die Lösung zur Injektion vorzugsweise für eine intravenöse Injektion, eine intraarterielle Injektion, eine intrathekale Injektion, oder eine intratumorale und peritumorale Injektion vorgesehen ist.

8. Pharmazeutische Lösung zur Verwendung nach Anspruch 1, wobei in dem Deuteriumoxid die Isotopenhäufigkeit von Deuterium 99,8 % bis 99,9 % beträgt.

9. Pharmazeutische Lösung zur Verwendung nach Anspruch 1, wobei die Dosis der Lösung zur Injektion 1 ml/kg bis 20 ml/kg beträgt.

10. Pharmazeutische Lösung zur Verwendung nach Anspruch 1, wobei der Tumor ausgewählt ist aus Lungenkrebs, kolorektalem Krebs, primärem Leberkrebs, Speiseröhrenkrebs, Magenkrebs und Herzkrebs, Bauchspeicheldrüsenkrebs, Nierenzellkarzinom, Blasenkrebs, Prostatakrebs, Kopf- und Halskrebs, Nasen-Rachen-Krebs, Gebärmutterhalskrebs, Eierstockkrebs, Brustkrebs, Hirntumor, Knochen- und Gelenkssarkom, Schilddrüsenkrebs, Hautkrebs, malignem Melanom, malignem Lymphom, Leukämie, und Komplikationen und Rezidiven von malignen Tumoren, wie etwa Rezidiven, Metastasen und Einnistungen von Tumorzellen in Hohlräumen von Brust, Bauchraum, Blase und/oder Becken sowie maligne Ergüsse in Hohlräumen von Brust, Bauchraum, Blase und/oder Becken.

**Revendications**

1. Solution pharmaceutique pour l'utilisation afin d'améliorer l'efficacité anti-tumorale et de réduire la toxicité d'un médicament anti-tumoral, lorsqu'elle est utilisée en combinaison avec au moins un médicament anti-tumoral dans le traitement ou la prévention des tumeurs malignes, dans laquelle la solution pharmaceutique contenant de l'oxyde de deutérium comme solvant, dans laquelle 100 ml de la solution pharmaceutique respectivement contiennent 95 à 99,9 ml de l'oxyde de deutérium, et l'abondance de l'isotope deutérium dans l'oxyde de deutérium est 90 % à 99,9 %, et dans laquelle le médicament anti-tumoral est choisi dans le groupe constitué par 5-fluorouracile, gemcitabine, mitomycine, oxaliplatine, paclitaxel, thiotépa et bevacizumab.

2. Solution pharmaceutique pour l'utilisation selon la revendication 1, **caractérisée en ce que** la solution pharmaceutique comprend du chlorure de sodium, étant une solution de chlorure de sodium et d'oxyde de deutérium contenant 0,1g à 5g du chlorure de sodium, de préférence 0,5 g à 2,5 g du chlorure de sodium pour 100 ml de la solution pharmaceutique, le plus préférablement étant une solution de chlorure de sodium et d'oxyde de deutérium contenant 0,9 g chlorure de sodium pour 100 ml solution pharmaceutique qui est préparée avec de l'oxyde de deutérium et du chlorure de sodium et a un pH réglé à 4,5 à 7,0.

3. Solution pharmaceutique pour l'utilisation selon la revendication 1, **caractérisée en ce que** la solution pharmaceutique comprend du glucose, étant une solution de glucose et d'oxyde de deutérium contenant 0,1 g à 50 g du glucose, de préférence 5 g à 50 g du glucose pour 100 ml de la solution pharmaceutique, le plus préférablement étant une solution de glucose et d'oxyde de deutérium contenant 5 ou 10 g du glucose pour 100 ml solution pharmaceutique qui est préparée avec de l'oxyde de deutérium et du glucose et a un pH réglé à 3,2 à 6,5.

4. Solution pharmaceutique pour l'utilisation selon la revendication 1, **caractérisée en ce que** la solution pharmaceutique est une solution de glucose, de chlorure de sodium et d'oxyde de deutérium contenant 0,9 g du chlorure de sodium et 5 g du glucose pour 100 ml de la solution pharmaceutique qui est préparée avec de l'oxyde de deutérium, du chlorure de sodium et du glucose et a un pH réglé à 3,5 à 5,5.

5. Solution pharmaceutique pour l'utilisation selon la revendication 1, **caractérisée en ce que** la solution pharmaceutique est une solution Ringer avec de l'oxyde de deutérium, contenant 0,9 g du chlorure de sodium, 0,012 g du chlorure de potassium et 0,024 g du chlorure de calcium pour 100 ml de la solution pharmaceutique qui est préparée avec de l'oxyde de deutérium, du chlorure de sodium, du chlorure de potassium et du chlorure de calcium et a un pH réglé à 4,5 à 7,5.

6. Solution pharmaceutique pour l'utilisation selon la revendication 1, **caractérisée en ce que** la solution pharmaceutique est une solution de bicarbonate de sodium dans de l'oxyde de deutérium, contenant 1 g à 50 g, de préférence 5 g à 10 g, le plus préférablement 5 g du bicarbonate de sodium pour 100 ml de la solution pharmaceutique et ayant un pH réglé à 7,5 à 8,5.

7. Solution pharmaceutique pour l'utilisation selon la revendication 1, dans laquelle la solution pharmaceutique étant une solution pour l'instillation et le lavage, une solution pour l'injection, une suspension, une émulsion, ou une solution pour l'embolisation ; et la solution pour l'injection de préférence étant prévue pour une injection intraveineuse, une injection intra-artérielle, une injection intrathécale ou une injection intratumorale et peritumorale.

8. Solution pharmaceutique pour l'utilisation selon la revendication 1, dans laquelle l'abondance de l'isotope deutérium dans l'oxyde de deutérium est 99,8 à 99,9 %.

9. Solution pharmaceutique pour l'utilisation selon la revendication 1, dans laquelle la dose de la solution pour l'injection est 1 ml/kg à 20 ml/kg.

10. Solution pharmaceutique pour l'utilisation selon la revendication 1, dans laquelle la tumeur est choisie parmi le cancer du poumon, le cancer colorectal, le cancer primaire du foie, le cancer de l'œsophage, le cancer gastrique et le cancer cardiaque, le cancer du pancréas, le carcinome des cellules rénales, le cancer de la vessie, le cancer de la prostate, le cancer de la tête et du cou, le cancer du nasopharynx, le cancer du col de l'utérus, le cancer des ovaires, le cancer du sein, la tumeur cérébrale, le sarcome des os et des articulations, le cancer de la thyroïde, le cancer de la peau, le mélanome malin, le lymphome malin, la leucémie, et les complications et la récidive de diverses tumeurs malignes, telles que la récidive, les métastases et l'implantation de cellules tumorales dans les cavités thoracique, péritonéale, vésicale et/ou pelvienne, et l'épanchement malin dans les cavités thoracique, péritonéale, vésicale et/ou pelvienne.

EP 3 150 212 B1

FIG.1

FIG.2

42

FIG.3

FIG.4

FIG.5

FIG.6

FIG.7

FIG.8

FIG.9

FIG.10

FIG.11

FIG.12

FIG.13

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006022460 A1

**Non-patent literature cited in the description**

- *CHEMICAL ABSTRACTS,* 7789-20-0
- **D.M. CZAJKA.** *Am. J. Physiol.,* 1961, vol. 201, 357-362
- *Science,* 1961, vol. 133, 1131-1133
- *Cancer Res.,* 1982, vol. 42, 1125-1129
- *Cancer,* 1988, vol. 62, 462-466
- *Int.J. Cancer.,* 1990, vol. 45, 475-480
- *Anticancer Res.,* 2005, vol. 25, 3407-3412
- *J.Ind. Eng.Chem.,* 2007, vol. 13, 501-507
- **BADER et al.** *Cancer Lett.,* 2008, vol. 259, 231-239